# EUROPÄISCHE PATENTSCHRIFT

(11) **EP 0 025 897 B1**
(45) Veröffentlichungstag und Bekanntmachung des Hinweises auf die Patenterteilung: **28.03.1984**
(21) Anmeldenummer: 80105158.2
(22) Anmeldetag: 29.08.1980
(51) Int. Cl.: C07C 103/52, A61K 37/02

(54) **Neue Peptide und Verfahren zu ihrer Herstellung**
Peptides and process for their preparation
Peptides et procédé pour leur préparation

(30) Priorität: 22.09.1979 DE 2938420
(43) Veröffentlichungstag der Anmeldung: 01.04.1981
(73) Patentinhaber: HOECHST AKTIENGESELLSCHAFT, 65926 Frankfurt am Main (DE)
(72) Erfinder: Geiger, Rolf, Prof. Dr., D-6000 Frankfurt am Main 50 (DE); König, Wolfgang, Dr., D-6238 Hofheim am Taunus (DE); Johnscher, Gerd, Dr., D-6233 Kelkheim (Taunus) (DE)

## Beschreibung

Aus Thymusextrakten wurden mehrere Peptide isoliert und in ihrer Struktur aufgeklärt, z. B. Thymosin α₁, Thymopoietin und der «facteur Thymique serique» (FTS), die unter anderem zur Differenzierung («Reifung») thymusabhängiger Lymphocyten (T-Zellen) beitragen.

Im Verlauf der Untersuchungen zur Abhängigkeit dieser Wirkung (die z.B. in vitro im Effekt auf autologe rosettenbildende Zellen aus der Milz thymusloser Mäuse analog Proc. Natl. Acas. Sci. U.S.A., 72 (1975), Seite 3201, erkennbar ist) von der Struktur niedermolekularer Peptide wurde nun gefunden, dass geeignete Peptide, die entsprechend den allgemeinen Eigenschaften ihrer Glieder wie folgt charakterisiert werden können:
basisch-basisch-sauer-hydrophob-hydrophob

alle in dem genannten und anderen Tests wirksam sind.

Gegenstand der Erfindung sind demnach Peptide der Formel 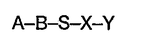 in welcher bedeutet
A. Arginin, Lysin, Ornithin oder Homoarginin, jeweils in L- oder D-Konfiguration,
   co-Amino-, -Guanidino- oder -Dimethylamino-alkanoyl mit 3 bis 6 C-Atomen und gegebenenfalls einer a-Aminogruppe, in D- oder L-Konfiguration, die ihrerseits Alkanoyl mit 1 bis 6 C-Atomen, Aroyl mit 7 bis 11 C-Atomen, Cycloalkanoyl mit bis zu 2 Alkyl- und 5 bis 7 Cycloalkyl-C-Atomen, Aralkanoyl mit bis zu insgesamt 9 C-Atomen, wobei eine-CH₂-Gruppe durch -O- oder-S- ersetzt sein kann, Alkyl- oder Aralkyl-oxycarbonyl mit bis zu 7 C-Atomen oder
   Succinoyl, Succinamoyl, Glutaroyl, Glutaminyl, Pyroglutamyl, Phthaloyl, Phthalamidyl oder 2-Carboxybenzoyl tragen kann,
B. eine basische Aminosäure, vorzugsweise L-Lysin, L-Arginin, L-Homoarginin oder L-Ornithin
   S. L-Glutaminsäure, D-Glutaminsäure, D-Asparaginsäure, D-a-Aminoadipinsäure
   X. L-Valin oder L-Isoleucin und
   Y eine Aminosäure mit hydrophober Seitenkette in L oder D-Konfiguration, deren Ester, Amid, Alkylamid mit 1 bis 6 C-Atomen oder Aralkylamid mit 7 bis 10 C-Atomen,

   Alkylamid oder Alkylester mit 1 bis 6 C-Atomen oder Aralkylamid oder Aralkylester mit 7 bis 10 C-Atomen.

Die saure Funktion in der oben wiedergegebenen Charakteristik wird erfindungsgemäss von der L- bzw. D-Glutaminsäure sowie von D-Asparaginsäure und D-a-Aminoadipinsäure wahrgenommen, der hydrophobe Bereich kann 1 bis 2 Aminosäuren oder deren Amide oder Ester umfassen, wobei S -Ile oder S -Val als zentraler Bereich den Peptiden besonders günstige Wirkqualität verleihen.

Der Substituent an der a-Aminogruppe von A und das Glied Y sind für die Wirkung nicht kritisch, sie beeinflussen sie jedoch in quantitativer Hinsicht. So kann insbesondere in A der Alkanoylrest ein Formyl- bis Hexanoylrest, der Aroylrest Benzoyl sein, das gegebenenfalls durch Methyl, Methoxy oder Chlor substituiert ist, der Aralkanoylrest Phenacetyl, Cinnamoyl, Dihydrocinnamoyl, Phenoxyacetyl oder Phenyithiacetyl, wobei der Arylrest unsubstituiert oder durch Methyl, Methoxy oder Chlor substituiert vorliegen kann.

Alkyl- oder Aralkyloxycarbonyl ist bevorzugt Ethyl-, Isobutyl-, tert.-Butyl-, Benzyl-, 4-Methylbenzyl-, 4-Methoxybenzyl oder 4-Chlorbenzyloxycarbonyl.

Die für Y stehende Aminosäure mit hydrophober Seitenkette kann insbesondere Ala, Val, Leu, lle, Met, Phe, Pro, Tyr, Phg (C-Phenylglycin) sein, ferner eine hydrophob substituierte Aminosäure wie Ser(Bu^{t}), Thr(Bu^{t}), Cys(Bu^{t}), Cys(Et), Cys(Bzi), Glu(OBu^{t}), Asp(OBu^{t}), Glu(NH-Bu^{t}), Glu(NH-Et), Lys(Boc), Orn(Boc), Tyr(Bu^{t}), Tyr(Me), Phe(CI), Tyr(CI), ferner alkyl-, halogen- oder methoxysubstituiertes Tryptophan. Als Alkylamide kommen vorzugsweise n-Alkyl- oder verzweigte Alkylamide wie Isopropyl-, Isobutyl-, tert.-Butyl-, 3-Methylbutyl- oder 3-Ethylbutylamid in Frage; als Aralkylamide z.B. Benzyl- oder Phenethylamid, die im Kern und/oder in der Seitenkette durch 1 bis 2 Methylgruppen substituiert sein können. Dieselben Alkyl-, Aryl- oder Aralkylreste können auch in Esterbindung vorliegen.

Die erfindungsgemässen Peptide besitzen Ähnlichkeit mit den aus der FR-A 2 408 580 bekannten Polypeptiden der Formel R-Arg-Lys-Asp-Val-Tyr-R' sowie mit einem kürzlich in Science 204 (1979), Seite 1309, beschriebenen Pentapeptid Arg-Lys-Asp-Val-Tyr, einer Teilsequenz des Thymopoietins, das als derjenige Bereich dieses Peptids angesprochen wird, der für die biologische Wirkung verantwortlich sein soll. Interessanterweise entspricht diese Sequenz der oben erwähnten Folge basisch-basisch-sauer-hydrophob-hydrophob, die damit als ein übergeordnetes Prinzip für Peptide der angeführten Thymusaktivität erscheint.

Im Gegensatz zu diesen Peptiden enthalten die erfindungsgemässen Peptide jedoch L- oder D-Glutaminsäure sowie D-Asparaginsäure oder D-a-Aminoadipinsäure als saure Aminosäure. Peptide der Glutaminsäure und a-Aminoadipinsäure sind in schwach saurem Milieu, in dem diese Verbindungen meist angewandt werden, wesentlich stabiler als Asparaginsäurepeptide. Letztere lagern sich über Asparaginimid-Peptide grösstenteils in Isoasparaginpeptide um, so dass z.B. eine Hitzesterilisation von Asparaginsäurepeptiden nicht möglich ist. Die Umlagerung erfolgt aber auch bei Raumtemperatur und selbst bei Kühlschranktemperatur noch mit merklicher Geschwindigkeit. Stehen für S die sauren D-Aminosäuren, so wird die enzymatische Stabilität der Peptide vergrössert.

Darüber hinaus umfassen die erfindungsgemässen Verbindungen auch Tri- und Tetrapeptidderivate, deren Wirkung in vielen Fällen gegenüber einem erfindungsgemäss hergestellten Pentapeptid der Sequenz Lys-Lys-Glu-Val-Val gleich oder sogar erhöht ist.

Gegenstand der Erfindung ist ferner ein Verfahren zur Herstellung der genannten Peptide, das dadurch gekennzeichnet ist, dass man Aminosäuresequenzen der Formel 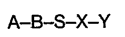 in der A, B, S, X und Y die im Anspruch 1 wiedergegebene Bedeutung haben, nach Methoden der Peptidsynthesen aufbaut.

Die Synthese der erfindungsgemässen Verbindungen folgt den bekannten Methoden der Peptidchemie, wie sie z.B. in Houben-Weyl, Methoden der Organischen Chemie, Band 15, ausführlich beschrieben sind. Die auf den folgenden Seiten wiedergegebenen Beispiele erläutern die an sich bekannten Syntheseverfahren.

Es werden die in der Peptidchemie gebräuchlichen Abkürzungen verwendet, insbesondere:

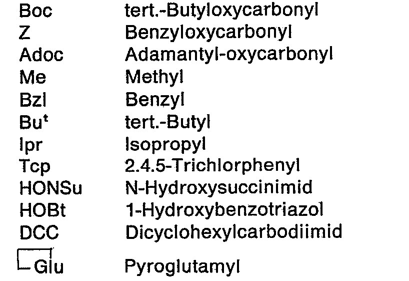

Ferner steht DC für Dünnschichtchromatographie, HPLC für Flüssigkeitschromatographie (high performance liquid chromatography). Bei den Aminosäureanalysen wird Glu = 100 gesetzt.

Die erfindungsgemässen Peptide besitzen in vitro in Anwesenheit von Leberhomogenaten gegenüber den natürlichen Thymuspeptiden eine wesentlich verlängerte Lebensdauer. Besonders hervorzuheben sind die Verbindungen, in denen für S eine saure D-Aminosäure wie D-Glutaminsäure oder D-a-Aminoadipinsäure steht.

Ihre Wirkung kann z. B. in vitro durch die Beeinflussung. von SRBC-Rosetten-bildenden T-Lymphocyten aus Blut von immundefizienten Patienten oder humanem Nabelschnurblut analog J. Exptl. Med. 136 (1972) Seite 207; Anm. N.Y. Acad. Sci. 249 (1975) Seite 308 und Int. Archs. Allergy appl. Immun. 53 (1977) Seite 242, sowie der PHAinduzierten Blastentransformation von humanen und animalischen Lymphocyten analog J. Exptl. Med. 131 (1970), Seite 1049, und Cell. Immunol. 16 (1975), Seite 413, nachgewiesen werden. (SRBC = sheep red blood cell; PHA = Phythämagglutinin).

Die erfindungsgemässen Verbindungen können zur Behandlung von Immundefizienzen, viralen und fungoiden, sowie chronisch bakteriellen Infekten. Autoimmunkrankheiten sowie zur Theraoie von Erkrankungen dienen, die durch Zellen mit immunologisch relevanten Veränderungen der Zellmembranmerkmale (z.B. Tumorzellen) verursacht werden.

In diesem Sinne ist Gegenstand der Erfindung auch die Verwendung der genannten Peptide ganz allgemein zur Beeinflussung der Reifung von T₋ Lymphozyten.

### Beispiel 1

### Arg-Arg-Glu-Val-Val-NH-Bzl, Acetat

28,8 g Adoc-Arg(Adoc)₂-Arg(Adoc)₂-Glu(OBu^{t})-OH, · 2N₂O, hergestellt nach Chem. Ber. 103 (1970), Seite 1727, und 6.8g H-Val-Val-NH-Bzl · HCI, hergestellt unter Verwendung von L-Valin als Aminosäure analog Ann. Chem. 655 (1962), Seite 211, werden in 120 ml Dimethylformamid gelöst. Man gibt 2.7 g HOBt und 2.6 ml N-Ethylmorpholin zu und trägt unter Rühren bei Raumtemperatur 5.0g DCC ein. Nach 15 Stunden filtriert man und destilliert das Lösungsmittel im Vakuum ab. Der Rückstand wird nacheinander mit Wasser, 1N Citronensäure, gesättigterNatriumbicarbonatlösung und Wasser verrieben und getrocknet. Zur Abspaltung der Schutzgruppen löst man in 25 ml Trifluoressigsäure und fällt nach 40 min mit Ether das rohe Pentapeptidderivat aus. Zunächst überführt man das erhaltene Trifluoracetat durch Rühren mit einem stark basischen Ionenaustauscher in der Acetatform in 50prozentigem Methanol in das Acetat, das nach Abfiltrieren des Austauschers und Abdestillieren des Lösungsmittels als harzig-halbfeste Masse vorliegt. Zur Reinigung löst man in 40 ml lprozentiger Essigsäure und chromatographiert über Sephadex^{(R)} LH-20 in einer Säule 4 x 200 cm. Die peptidhaltigen Fraktionen werden dünnschichtchromatographisch kontrolliert, zusammengefasst und lyophilisiert. Ausbeute an DC-einheitlichem Peptid-benzylamid-acetat 10.4 g. Aminosäureanalyse: Glu 1.00, Val 1.87, Arg 1.99. In der Dansyl-Endgruppenbestimmung wurde kein Valin gefunden.

### Beispiel 2

### Arg-Arg-Glu-Val-Phe-OMe, Acetat

Man setzt analog Beispiel 1) 28.8 g Adoc-Arg-(Adoc)₂-Arg(Adoc)₂-Glu(Obu^{t})-OH · 2.5 H_{z}0 mit 6.3 g H-Val-Phe-OMe · HCI, hergestellt nach J. Amer. Chem. Soc. 84 (1962), Seite 2417, um und erhält nach Abspaltung der Schutzgruppen und Reinigung 9.4 g Peptid, Aminosäureanalyse: Glu 1.00, Val 0,97, Phe 1.01, Arg 2.04.

### Beispiel 3

### Arg-Arg-Glu-Val-Tyr-NH_{z}, Acetat

Man verfährt nach Beispiel 1, setzt jedoch 5.6 g H-Val-Tyr-NH_{z}, hergestellt nach Chem. Ber. 97 (1964), Seite 1197, ein und erhält nach Abspalten der Schutzgruppen und Reinigung 9.0 g der Titelverbindung als Acetat.

Aminosäureanalyse: Glu 1.00, Val 0,96, Tyr 0.88, Arg 2.02.

### Beispiel 4

### Arg-Arg-Glu-Ile-Cys(Bzl)-OEt, Acetat

Man spaltet von Z-Ile-Cys(Bzl)-OEt, hergestellt nach J. Amer. Chem. Soc. 83 (1961), Seite 145, durch 30 min Behandeln mit HBr/Eisessig die Benzyloxycarbonylgruppe ab und erhält nach Fällen mit Ether und zweimaligem Umfällen aus EtOH/Ether und Trocknen im Exsiccator über KOH als kristalline, etwas hygroskopische Masse das Hydrobromid H-Ile-Cys (Bzl)-OEt · HBr. Man setzt 7.0 g dieser Verbindung mit 2.88 g der Carboxylkomponente analog Beispiel 1 um und erhält nach Abspalten der Schutzgruppen und Reinigung 11.2 g der Titelverbindung als Acetat. Aminosäureanalyse: Glu 1.00, Ite 0.91, Cys(Bzl) 0.92, Arg 2.01.

### Beispiel 5

### Arg-Arg-Glu-Val-Val-O-CH(CH₃)_{z}, Acetat

### A) Boc-Val-Val-Olpr

5.38 g H-Val-Olpr · HCl, in bekannter Weise durch Veresterung von L-Valin mit HCI/Isopropanol hergestellt, werden unter Zusatz von 4.0 ml N-Ethylmorpholin in 20 ml Dimethylacetamid gelöst. Gleichzeitig stellt man symmetrisches Boc-Valanhydrid aus 13.1 g Boc-Val-OH und 6.6 g DCC in 50 ml Dimethylacetamid bei 0° her. Man vereinigt beide Lösungen, lässt auf Raumtemperatur kommen, rührt noch 4 Stunden bei etwa 22° und destilliert das Lösungsmittel im Vakuum ab. Der Rückstand wird in 150 ml Essigester aufgenommen und dreimal mit je 20 ml Wasser ausgeschüttelt.

Nach Trocknen über Natriumsulfat wird der Essigester abdestilliert und der Rückstand aus Hexan umkristallisiert. Ausbeute 6.9 g, Schmp. 126-129°C; Elementaranalyse (C,H,N) korrekt.

### B) H-Val-Val-Olp, CF₃COOH

Die Boc-Verbindung wird in 60 ml Trifluoressigsäure gelöst. Nach 40 min fällt man mit Ether/ Petrolether (1 : 1) einen Niederschlag aus, der mit demselben Gemisch gründlich digeriert wird. Nach dem Trocknen im Vakuum über KOH ist die Ausbeute 6.1 g, Schmp. 172-174°C. Elementaranalyse (N,F) korrekt.

### C) Arg-Arg-Glu-Val-Val-Olpr, Acetat

Man setzt 3.7 g der nach B) gewonnenen Verbindung mit 14.4 g Adoc-Arg(Adoc)₂-Arg(Adoc)₂-Glu(OBu^{t})-OH · 2 H₂O, 1.35 g HOBt, 1.3 ml N-Ethylmorpholin und 2.5 g DCC analog Beispiel 1 um und erhält nach Anspalten der Schutzgruppen und Reinigung 5.8 g Peptid. Aminosäureanalyse: Glu 1.00, Val 1.90, Arg 2.07.

### Beispiel 6

### Arg-Arg-Glu-Val-Lys(Z)-OMe, Acetat

### A) Boc-Val-Lys(Z)-OMe

4.35 g Boc-Val-OH und 6.6 g H-Lys(Z)-OMe, HCI werden in 50 ml CH₂Cl₂ unter Zusatz von 2.7 g HOBt und 2.6 ml N-Ethylmorpholin (NEM) mit 4.4 g DCC kondensiert. Nach Filtration wird das Lösungsmittel abdestilliert. Der Rückstand wird in Essigester aufgenommen, nacheinander mit 2N Citronensäure, 1M Natriumhydrogencarbonat und Wasser gewaschen, über Natriumsulfat getrocknet und nach Abdestillieren des Essigesters aus Diisopropylether umkristallisiert. Ausbeute 3.5 g, Schmp. 100-102°, Elementaranalyse (C,H,N) korrekt.

### B) H-Val-Lys(Z)-OMe, CF₃COOH

Der Boc-Rest wird analog Beispiel 5 B) abgespalten. Ausbeute 3.1 g. In der DC fast einheitlich.

### C) Arg-Arg-Glu-Val-Lys(Z)-OMe, Acetat

Umsatz von 2.5 g der nach B) erhaltenen Verbindung mit 7.2 g Carboxylkomponente, 0,65 ml NEM, 0.7 g HOBt und 1.1 g DCC analog Beispiel 1. Nach Abspalten der Schutzgruppen und Reinigung analog Beispiel 1 erhält man 3,2 g der Titelverbindung als Acetat. Aminosäureanalyse: Glu 1.00, Val 0.97, Lys 1.02, Arg 1.99.

### Beispiel 7

### Arg-Arg-Glu-Ile-Olpr, Acetat

Man stellt aus Isoleucin und HCI/Isopropanol in bekannter Weise Isoleucin-isopropylester-hydrochlorid her. 2.1 g dieser Verbindung werden analog Beispiel 5 C) mit 14.4 g Adoc-Arg(Adoc)_{z-}Arg-(Adoc)₂-Glu(OBu^{t})-OH · 2 H₂O umgesetzt und aufgearbeitet. Ausbeute 6.2 g Aminosäureanalyse: Glu 1.00, Ile 0.95, Arg 2.06.

### Beispiel 8

### Glu-Arg-Arg-Glu-Ile-Olpr, Acetat

8.5 g der nach Beispiel 7 erhaltenen Verbindung werden in 50 ml Dimethylformamid mit 3.5 g L-Pyroglutaminsäure-trichlorphenylester in Anwesenheit von 0.2 g HOBt umgesetzt. Nach 4 Stunden fällt man mit Essigester ein Rohprodukt aus, das durch HPLC an SiO₂ im System Chloroform-Methanol-Essigsäure (3:2:1) gereinigt wird. Ausbeute 5.3 g. Aminosäureanalyse: Glu 2.00, lle 0.92, Arg 1.98.

### Beispiel 9

### Z-Arg-Arg-Glu-Val-Val-NH₂, Acetat

### A) H-Val-Val-NH₂ · HCI

7.53 g Z-Val-OH, 5.91 g H-Val-NH₂ · HBr und 4.05 g HOBt werden in 40 ml Dimethylformamid gelöst. Dazu gibt man 3.9 ml N-Ethylmorpholin und bei 0°C 6.6 g DCC. Der Ansatz wird 1 Stunde bei 0° und 3 Stunden bei Raumtemperatur gerührt. Es entsteht ein dicker weisser Brei, der mit 150 ml Wasser und 50 ml gesättigter NaHCO₃-Lösung verrührt wird. Der Niederschlag wird abgesaugt und über P₂O₅ im Vakuum getrocknet. Ausbeute 16.6 g. Die Substanz ist eine Mischung aus Z-Val-Val-NH₂ und Dicyclohexylharnstoff. 14.85 g der so gewonnenen Substanz werden in 40 ml Eisessig suspendiert. Nach Zugabe von 40 ml 4n HBr/Eisessig geht die Substanz in Lösung. Man lässt eine Stunde bei Raumtemperatur stehen und fällt mit 400 ml Äther einen schmierigen Niederschlag aus. Man dekantiert ab und löst den Rückstand in 60 ml Methanol. Mit 200 ml Ether wird eine weisse, kristalline Substanz gefällt. Nach Trocknen über P₂O₅ beträgt die Ausbeute 6.7 g, Schmp. 274-278°C, [α]D²³ = + 5.9° (c = 1, in Methanot).

### B) Z-Glu(OBu^{t})-Val-Val-NH₂

Zu einer Lösung von 6 g H-Val-Val-NH₂ · HBr, 2.7 g HOBt und 2.6 ml N-Ethylmorpholin in 20 ml Dimethylformamid gibt man 10.4g Z-Glu(OBu^{t})-OTcp. Der Ansatz wird sofort gallertig. Man verdünnt mit 75 ml Dimethylformamid und rührt noch 2 Stunden. Dann wird der Ansatz in 900 ml Wasser eingerührt, das 50 ml gesättigte NaHCO₃-Lösung enthält. Der Niederschlag wird abgesaugt, mit etwa 200 ml Essigester verrieben, erneut abgesaugt und nachgewaschen mit Essigester. Ausbeute 6.55 g, Schmp. 256-257°.

### [α]_{D}²³ = + 4_{.7}° (c = 1, in Dimethylacetamid)

### C) H-Glu(OBu^{t})-Val-Val-NH₂ · HCI

6 g Z-Glu(OBu^{t})-Val-Val-NH₂ werden in 200 ml Methanol suspendiert. Unter Zusatz von Pd/ BaSO₄ und Zugabe von 2N methanolischer Salzsäure wird bei pH 4.5 katalytisch hydriert. Nach beendigter Reaktion wird der Katalysator abgesaugt und das Filtrat eingeengt. Der Rückstand kristallisiert beim Verreiben mit Ether. Ausbeute 4.8 ^{g.} Schmp. 211-219°, [α]_{D}²³ = - 18.7° (C = 1, in Methanol).

### D) Z-Arg-Arg-Glu-Val-Val-NH_{"}, Acetat

6.3 g Z-Arg-Arg-OH - 2 HBr, hergestellt nach Experientia (Basel) 12, (1956), Seite 446, und 4.4 g der unter C) erhaltenen Verbindung werden in 50 ml Dimethylformamid gelöst. Man gibt unter Rühren nacheinander 1.35g HOBt, 1.3 ml N-Ethylmorpholin und 2.2 g DCC zu und rührt noch 5 Stunden. Dann wird filtriert und das Filtrat im Vakuum eingeengt. Abspaltung der tert. Butylestergruppe mit Trifluoressigsäure, Überführen in das Acetat und Reinigung erfolgt analog Beispiel 1. Ausbeute 4.5 g. Aminosäureanalyse: Glu 1.00, Val 1.88, Arg 2.02.

### Beispiel 10

### D-Arg-Lys-Glu-Val-Val-OMe, Acetat

### A) H-Val-Val-OMe . HCI

30 g Z-Val-Val-OMe (hergestellt nach Chem. Ber. 103, 788-798 (1970)) werden in 250 ml Methanol gelöst und bei pH 4 unter Zugabe von methanolischer Salzsäure und Zusatz von Pd/BaSO₄ katalytisch hydriert. Nach beendigter Reaktion wird der Katalysator abgesaugt und das Filtrat eingeengt. Das resultierende Öl wird mit Ether verrieben. Nach einiger Zeit kristallisiert die Substanz. Man saugt ab und wäscht mit Ether nach. Die Substanz ist hygroskopisch und wird über P₂O₅ im Vakuum getrocknet. Ausbeute 18.8 g, Schmp. 153°. [α]_{D}²³ + 2.5° (c = 1, in Methanol).

### B) Z-Glu(OBut)-Val-Val-OMe

Zu einer Lösung von 13.3 g H-VaIVaI-OMe · HCI und 7.75 g HOBt in 50 ml Dimethylformamid gibt man 6.5 ml N-Ethylmorpholin und unter Rühren 25.85 g Z-Glu(OBu^{t})-OTcp. Nach drei Stunden wird der Ansatz mit 300 ml Wasser und 100 ml gesättigter NaHCO₃-Lösung verrührt. Der ausfallende Niederschlag wird abgesaugt und in 100 ml Essigester gelöst. Die Essigesterphase wird jeweils einmal mit 50 ml K₂SO₄/KHSO₄-Lösung, 50 ml gesättigter NaHCO₃-Lösung und Wasser ausgeschüttelt, über Na₂SO₄ getrocknet und eingeengt. Der Rückstand wird mit Petroläther verrieben und abgesaugt. Ausbeute 23.6 g, Schmp. 138-142°. Zur weiteren Reinigung wird aus 100 ml Essigester umkristallisiert. Ausbeute 13.25g, Schmp. 173°, [α]_{D}²³ = - 42.4° (c = 1, in Methanol).

### C) H-Glu(OBu^{t})-Val-Val-OMe · HCI

13 g Z-Glu(OBu^{t})-Val-Val-OMe werden in etwa 150 ml Methanol suspendiert. Unter Zusatz von Pd/BaSO₄ und Zugabe von 2N methanolischer Salzsäure wird bei pH 4.5 katalytisch hydriert. Nach beendeter Reaktion wird der Katalysator abgesaugt und das Filtrat eingeengt. Der Rückstand kristallisiert beim Verreiben mit Ether. Ausbeute 9.55 g, Schmp. 192°, [α]_{D}²³ = - 20.9° (c = 1, in Methanol).

### D) Z-Lys(Boc)-Glu(OBu^{t})-Val-Val-OMe

Zu einer Lösung von 4.52 g H-Glu(OBu^{t})-Val-Vai-OMe · HCI, 1.35 g HOBt und 1.3 ml N-Ethylmorpholin in 10 ml Dimethylformamid gibt man eine Lösung von 5.6 g Z-Lys(Boc)-OTcp in 15 ml Dimethylformamid. Man rührt zwei Stunden bei Raumtemperatur und versetzt die Lösung mit 200 ml Wasser und 25 ml gesättigter NaHCO₃-Lösung. Der Niederschlag wird abgesaugt und in 100 ml Essigester gelöst. Wasser wird im Scheidetrichter abgetrennt. Die Essigesterphase wird mit Na₂SO₄ getrocknet und auf etwa 50 ml eingeengt. Dazu gibt man 200 ml Petroläther, kühlt auf 0°C und saugt ab. Ausbeute 7.6 g, Schmp. 171-177°, [α]_{D}²³ = - 36.6° (c = 1, in Methanol).

### E) H-Lys(Boc)-Glu(OBu^{t})-Val-Val-OMe · HCl

7 g Z-Lys(Boc)-Glu(OBu^{t})-Val-Val-OMe werden in 150 ml Methanol gelöst. Unter Zusatz von Pd/ BaSO₄ und Zugabe von 2N methanolischer Salzsäure wird bei pH 4.5 katalytisch hydriert. Nach beendeter Reaktion wird der Katalysator abgesaugt und das Filtrat eingeengt. Der Rückstand kristallisiert beim Verreiben mit Ether. Ausbeute 5.45 g, Schmp. 194-198°, [α]_{D}²³ = - 33,1° (c = 1, in Methanol).

### F) D-Arg-Lys-Glu-Val-Val-OMe, Acetat

1.37 g Boc-D-Arg-OH und 3.4 g der nach E) erhaltenen Verbindung werden in 30 ml Dimethylformamid gelöst. Man gibt 0.68 g HOBt und 1.05 g DCC zu und rührt über Nacht. Dann wird filtriert und das Filtrat im Vakuum eingeengt. Der Rückstand wird zur Abspaltung der Schutzgruppen in 20 ml Trifluoressigsäure aufgenommen. Nach 30 Minuten fällt man das rohe Peptid mit Ether, überführt es analog Beispiel 1 in das Acetat und reinigt es durch Chromatographie an Sephadex^{lR}) LH-20. Ausbeute 3.0 g. Aminosäureanalyse: Glu 1.00, Val 1.92, Lys 0.98, Arg 1.01.

### Beispiel 11

### Z-Arg-Arg-Glu-Val-Val-OMe, Acetat

### A) Z₃-Arg-Glu(OBu^{t})-Val-Val-OMe

Zu einer Lösung von 4.52 H-Glu(OBu^{t})-Val-Val-0Me · HCI und 1.35 g HOBt in 10 ml Dimethylformamid gibt man 1.3 ml N-Ethylmorpholin und 7.6 g Z₃-Arg-OTcp, gelöst in 15 ml Dimethylformamid. Nach 2 Stunden verrührt man die Reaktionsmischung mit 200 ml Wasser und 25 ml gesättigter NaHCO₃-Lösung. Der Niederschlag wird abgesaugt und mit Wasser gewaschen. Aus dem Dampfbad wird die noch feuchte Substanz in,400 ml Essigester gelöst. Die Essigesterlösung wird mit Na₂SO₄ getrocknet und auf etwa 100 ml eingeengt. Dazu gibt man 200 ml Petroläther, kühlt kurz, saugt ab und wäscht mit Petroläther. Ausbeute 8.6 g, Schmp. 204-209°, [α]_{D}²³- 2.4° (c = 1, in Dimethylacetamid).

### B) H-Arg-Glu(OBu^{t})-Val-Val-OMe · 2HCI

8 g Z₃-Arg-Glu(OBu^{t})-Val-Val-OMe werden in 150 ml Methanol und 40 ml Dimethylformamid suspendiert und nach Zusatz von Pd/BaSO₄ und unter Zugabe von 2N methanolischer Salzsäure bei pH 4.5 katalytisch hydriert. Nach beendeter Reaktion wird der Katalysator abgesaugt und das Filtrat eingeengt. Der Rückstand wird mit Ether verrieben. Der Ether wird abdekantiert und das Öl im Hochvakuum getrocknet. Ausbeute 5.1 g amorpher Schaum.

### C) Z-Arg-Arg-Glu-Val-Val-OMe, Acetat

1.54 g Z-Arg-OH und 3.44 g der nach B) hergestellten Verbindung werden in 40 ml Dimethylformamid nacheinander mit 0.68 g HOBt und 1.1 g DCC versetzt. Nach Rühren über Nacht wird filtriert, das Filtrat im Vakuum eingeengt und der Rückstand mit Essigester digeriert.

Man spaltet analog Beispiel 1 die tert.-Butylgruppe mit Trifluoressigsäure ab, überführt ins Acetat und reinigt die Verbindung durch Chromatographie an Sephadex^{lR}) LH 20. Ausbeute 2.9 g, Aminosäureanalyse: Glu 1.00, Val 1.89, Arg 2.01.

### Beispiel 12

### Z-Lys-Arg-Glu-Val-Val-OMe, Acetat

6.4 g H-Arg-Glu(OBu^{t})-Val-Val-OMe, 2 HCI, hergestellt nach Beispiel 11 B), werden zusammen mit 4.0 g Z-Lys(Boc)-ONSu, 1.28 ml N-Ethylmorpholin und 1.35 g HOBt in 80 mi Dimethyiacetamid gelöst. Man versetzt mit 2.2 g DCC, rührt über Nacht, filtriert und engt das Filtrat im Vakuum ein. Der Rückstand wird mit Essigester digeriert. Abspaltung der tert.-Butylschutzgruppen, Überführen ins Acetat und Reinigung an Sephadexl^{R}) LH 20 erfolgt analog Beispiel 1. Ausbeute 5.8 g. Aminosäureanalyse: Glu 1.00, Val 1,90, Lys 1.03, Arg 1.00.

### Beispiel 13

### D-Lys-Arg-Glu-Val-Val-OMe, Acetat

Man verfährt nach Beispiel 12, setzt jedoch Z-D-Lys(Boc)-ONSu ein. Das Rohprodukt wird in 90prozentiger Essigsäure an Pd katalytisch hydriert. Sodann verfährt man zur Abspaltung von Boc und Bu^{t}, zur Überführung in das Acetat und nachfolgenden Reinigung analog den Beispielen 12 bzw. 1. Ausbeute 6.0 g. Aminosäureanalyse: Glu 1.00, Val 1.89, Lys 0.99, Arg 1.02.

### Beispiel 14

### Ethyloxycarbonyl-Lys-Lys-Giu-Val-Val-OMe, Acetat

6.8 g H-Lys(Boc)-Glu(OBu^{t})-Val-Val-OMe · HCI, hergestellt nach Beispiel 10 E), 4.0 g Z-Lys(Boc)-ONSu und 1.28 ml N-Ethylmorpholin werden in 70 ml Dimethylformamid über Nacht gerührt. Man destilliert das Lösungsmittel im Vakuum ab und nimmt den Rückstand in feuchtem Essigester n-Butanol (10+1) auf. Nach Waschen mit gesättigter NaHCO₃-Lösung und Wasser trocknet man über Natriumsulfat und destilliert das Lösungsmittel im Vakuum ab. Der harzige Rückstand wird in 100 ml Methanol gelöst und unter Titration mit 2N HCI in Methanol bei pH 4 katalytisch an Pd hydriert. Nach Abfiltrieren des Katalysators bringt man zur Trokkene, nimmt den festen Rückstand in 50 ml Dimethylformamid auf und gibt 4,0 g Kohlensäureethyl-pentachlorphenylester (Bull. Soc. Chim. France 23 (1900), Seite 818) zu. Man rührt über Nacht, destillierte das Lösungsmittel im Vakuum ab und digeriert den Rückstand mit Ether. Zum Abspalten der Schutzgruppen, Überführen ins Acetat und zur Reinigung wird analog Beispiel 1 verfahren. Ausbeute 4.1 g. Aminosäureanalyse: Glu 1.00, Val 1.92, Lys 2.02.

### Beispiel 15

### 2-Carboxy-benzoyl-Arg-Arg-Glu-Val-Val-Olpr, Acetat

8.2 g H-Arg-Arg-Glu-Val-Val-Olpr, Acetat (Beispiel 5 C) und 1.5 g Phthalsäureanhydrid werden in 50 ml N-Methylpyrrolidon/Pyridin 2: 1 gelöst. Man gibt 1.35 g HOBt zu und rührt 4 Stunden. Dann wird mit Ether ein Rohprodukt ausgefällt, das nach einmaligem Umlösen aus Methanol/Essigester dünnschichtchromatographisch fast einheitlich ist. Nebenprodukte unter 5%. Ausbeute 6.9 g. Aminosäureanalyse wie Ausgangsprodukt. Nach der Dansylmethode ist keine freie a-Aminogruppe zu erkennen.

### Beispiel 16

### Succinoyl-Arg-Arg-Glu-Val-Val-Olpr, Acetat

Man verfährt nach Beispiel 15, setzt jedoch anstelle des Phthalsäureanhydrids 1.1 g Bernsteinsäureanhydrid ein und arbeitet wie unter Beispiel 15 beschrieben auf. Ausbeute 6.1 g. Nach der Dansylmethode ist keine freie a-Aminogruppe zu erkennen.

### Beispiel 17

### Cyclohexylacetyl-Arg-Arg-Glu-Val-Val-NH-Bzl, Acetat

Man tropft die Lösung von 1.5 g Cyclohexylacetylchlorid in 5 ml Methylenchlorid bei 0-4°C unter Rühren zur Lösung von 7.1 g H-Arg-Arg-Glu-Val-Val-NH-Bzl, Acetat, hergestellt nach Beispiel 1, in 40 ml Dimethylacetamid, lässt 1 Stunde nachreagieren und fällt das rohe Reaktionsprodukt mit Ether aus. Abspaltung der Boc-Gruppe analog der von Adoc in Beispiel 1. Man überführt, wieder analog Beispiel 1, in die Acetatform und reinigt durch Chromatographie an Sephadex^{(R)} LH 20. Ausbeute 4.8 g. Aminosäureanalyse: Glu 1.00, Val 1.88, Arg 1.97.

### Beispiel 18

### ε-Aminocaproyl-Arg-Glu-Val-Val-OMe,

### Acetat

Man stellt aus 2.31 g Boc-s-Aminocapronsäure, 1,15 g HONSu und 2.02 g DCC in 10 ml Dimethylformamid den Aktivester her, filtriert vom ausgefallenen Dicyclohexylharnstoff ab, vereinigt das Filtrat mit der Lösung von 5.2 g Arg-Glu(OBu^{t})-Val-Val-OMe, 2HCI, (Beispiel 11 B) in 25 ml Dimethylformamid und gibt 1.03 ml N-Ethylmorpholin zu. Nach Stehen über Nacht wird das Lösungsmittel im Vakuum abdestilliert und der Rückstand mit Essigester digeriert. Abspalten der Schutzgruppe und Reinigung erfolgt analog Beispiel 1. Ausbeute 3.6 g, Aminosäureanalyse: Glu 1.00, Val 1.90, s-Cap 1.03, Arg 1.01.

### Beispiel 19

### s-Guanidocaproyl-Arg-Glu-Val-Val-OMe, Acetat

2 g der nach Beispiel 18 erhaltenen Verbindung werden in 10 ml Wasser gelöst. Man versetzt mit der Lösung von 11 g O-Methylisoharnstoff-hydrochlorid in 30 ml Wasser und hält den pH-Wert der Lösung mit 2N NaOH bei 9. Nach 20 Stunden wird ein Teil des Wassers im Vakuum abdestilliert. Die resultierende konzentrierte Lösung wird über eine Säule Biogel^{(R)} P2 (4x200 cm) entsalzt, wobei gleichzeitig das Peptid gereinigt wird. Ausbeute 1.2 g. Zur weiteren Reinigung wird das Peptid an den stark sauren Ionenaustauscher Lewatit S 100 in einer Säule 2 x 50 cm adsorbiert und mit 0.2N Ammoniak fraktioniert eluiert. Die Konzentrationsänderung im Eluat durch die austretenden Peptide wird über den Brechungsindex ermittelt (Differentialrefraktometer). Ausbeute nach Lyophilisieren der Fraktion, Wiederauflösen in 5prozentiger Essigsäure und erneutem Lyophilisieren 0.6 g. Aminosäureanalyse: Glu 1.00, Val 1.88, s-Guanidocaproyl nicht bestimmt, ε-Aminocaproyl < 0.02, Arg 1.01.

### Beispiel 20

### N-ε-Dimethyl-D-lysyl-Lys-Glu-Val-Val-OMe, Acetat

Man setzt 6.8 g H-Lys(Boc)-Glu(OBu^{t})-Val-Val-OMe · HCl, erhalten nach Beispiel 10, mit 4,0 g Boc-B-Lys(Z)-ONSu analog Beispielen 10 und 12 um und erhält nach katalytischer Hydrierung in 50 ml Methanol in Anwesenheit von 5 mi 30prozentigem Formaldehyd 7 g rohes Boc-D-Lys(CH₃)₂-Lys (Boc)-Glu(OBu^{t})-Val-Val-OMe. Abspalten der Schutzgruppen mit Trifluoressigsäure, Überführen ins Acetat und Reinigung erfolgt analog Beispiel 1. Ausbeute 2.9 g. Aminosäureanalyse: Glu 1.00, Val 1.90, Lys 1.01, N^{ε}-Dimethyllysin 0.93.

### Beispiel 21

### Lys-Arg-Glu-Val-Thr(Bu^{t})-OBu^{t}, Acetat

### A) H-Val-Thr(Bu^{t})-OBu^{t} · HCI

Man setzt 25.1 g Z-Val-OH und 23.1 g H-Thr(Bu^{t})-OBu^{t} in 200 ml Tetrahydrofuran in Anwesenheit von 13.5 g HOBt mit 22 g DCC um, filtriert nach Rühren über Nacht, destilliert das Lösungsmittel ab, nimmt den Rückstand in 300 ml Essigester auf, wäscht nacheinander mit 2N Citronensäure (0°C), gesättigtem Natriumhydrogencarbonat und Wasser, trocknet über Natriumsulfat und destilliert den Essigester nach Filtration ab. Der Rückstand wird in 400 ml Methanol gelöst. Man hydriert katalytisch an Pd unter Titration mit 2N methanolischer HCI bei pH 4, filtriert nach beendeter Reaktion vom Katalysator ab und bringt die Lösung zur Trockene. Der feste Rückstand wird mit Ether digeriert und im Vakuum getrocknet. Schmp. 135-140° (Z). Elementaranalyse (C,H,N,CI) korrekt. Ausbeute 28.3 g.

### B) Z-Glu(OBzl)-Val-Thr(Bu^{t})-OBu^{t}

25.7 g H-Val-Thr(Bu^{t})-OBu^{t} HCI werden in 250 ml Dimethylformamid mit 26.0 g Z-Glu(OBzl)-OH und 15 g DCC in Anwesenheit von 9.5 HOBt umgesetzt. Nach 6stündigem Rühren wird filtriert und das Filtrat im Vakuum zur Trockene gebracht. Man nimmt den Rückstand in Essigester auf, wäscht die Lösung wie unter A) und erhält nach Trocknen und Eindampfen des Lösungsmittels 37.3 g der Titelverbindung vom Schmp. 140-144° (Zers.). Elementaranalyse korrekt (C,H,N).

### C) Glu-Val-Thr(Bu^{t})-OBu^{t}

Die nach B) erhaltene Verbindung wird in 80prozentigem Methanol katalytisch an Pd hydriert. Man filtriert vom Katalysator ab und bringt die Lösung im Vakuum zur Trockene. Verreiben des Rückstandes mit Ether führt zur chromatographisch reinen Verbindung.

### D) Lys-Lys-Glu-Val-Thr(Bu^{t})-OBu^{t}, Acetat

6.8 g Z-Lys(Z)-Lys(Z)-OH, erhalten nach Chem. Ber. 93 (1960), Seite 2387, werden in 30 ml Dimethylacetamid mit 1.35 g HOBt und 2.06 g DCC voraktiviert. Die filtrierte Lösung wird mit der Lösung von 4.6 g der nach C) erhaltenen Verbindung in 30 ml N-Methylpyrrolidon vereinigt. Das Lösungsmittel wird nach 15 Stunden im Vakuum abdestilliert, der Rückstand in 90prozentiger Essigsäure aufgenommen und katalytisch an Pd hydriert. Man erhält nach Abfiltrieren des Katalysators, Abdestillieren des Lösungsmittels, Digerieren des Rückstandes mit Ether und Trocknen ein Rohprodukt, das durch Chromatographie an Silicagel im System Chloroform : Methanol : Essigsäure : Wasser (3 : 3 :1 :1) gereinigt wird. Ausbeute '6.1 g Aminosäureanalyse: Glu 1.00, Thr 0,89, Val 0,92, Lys 2.05.

### Beispiel 22

### Lys-Lys-Glu-Val-Val-OBu^{t}, Acetat

### A) H-Val-Val-OBu^{t} · HCl

37.7 g Z-Val-OH, 31.4 g H-Val-OBu^{t} · HCI und 19.75 g HOBt werden in 200 ml Dimethylformamid gelöst. Dazu gibt man 19.5 ml N-Ethylmorpholin und bei 0 °C 33 g DCC. Man rührt 1 Stunde bei 0 °C und 1 Stunde bei Raumtemperatur, saugt den Niederschlag ab und engt das Filtrat im Vakuum ein. Der Rückstand wird zwischen 200 ml Essigester und 200 ml Wasser verteilt. Die Essigesterphase wird danach mit je 150 ml gesättigter NaHCO₃-Lösung, K₂SO₄/KHSO₄-Lösung, gesättigter NaH-CO₃-Lösung und Wasser ausgeschüttelt, über Na₂SO₄ getrocknet und eingeengt. Das resultierende Öl (60 g) wird in 300 ml Methanol gelöst und bei pH 4.5 unter Zugabe von methanolischer Salzsäure und Zusatz von Pd/BaSO₄ katalytisch hydriert. Nach beendeter Reaktion wird der Katalysator abgesaugt und das Filtrat eingeengt. Der Rückstand kristallisiert aus Ether. Ausbeute 40 g. Schmp. 191-194, [α]_{D}²³ = -8.3° (c = 1, in Methanol).

### B) H-Glu-Val-Val-OBu^{t}

31 g H-Val-VaI-OBu^{t} · HCI werden analog Beispiel 21 B) mit 37 g Z-Glu(OBzl)-OH in Dimethylformamid in Anwesenheit von 13.5 g HOBt und 12.8 ml N-Ethylmorpholin mittels 22 g DCC kondensiert. Nach Aufarbeitung analog diesem Beispiel wird wie in Beispiel 21 C) beschrieben katalytisch hydriert. Die in Ether unlösliche Verbindung wird isoliert und ist dünnschichtchromatographisch einheitlich. Ausbeute 51 g.

### C) Lys-Lys-Glu-Val-Val-OBu^{t}, Acetat

Man aktiviert 6.8 g Z-Lys(Z)-Lys(Z)-OH Analog Beispiel 21 D) vor, setzt die erhaltene Lösung mit der Lösung von 4.2 g der nach B) erhaltenen Verbindung in 30 ml Dimethylformamid um und arbeitet wie in Beispiel 21 D) beschrieben auf. Ausbeute 5.85 g Aminosäureanalyse: Glu 1.00, Val 1.87, Lys 1.99.

### Beispiel 23

### Lys-Lys-Glu-Ile-Val-OBu^{t}, Acetat

### A) Z-Ile-Val-OBu^{t}

163.7 g Z-Ile-OH-Dicyclohexylammoniumsalz werden unter Rühren zwischen 600 ml Essigester und 370 ml IN Schwefelsäure verteilt. Die Essigesterphase wird mit Wasser extrahiert, über Na₂SO₄ getrocknet und eingeengt. Der Rückstand wird mit 73.15 g H-Val-OBu^{t} · HCI und 47.25 g HOBt in 350 ml Dimethylformamid gelöst. Dazu gibt man 44.8 mf N-Ethylmorpholin und bei 0°C eine Lösung von 73.5 g DCC in 100 ml Dimethylformamid. Man rührt 1 Stunde bei 0°C und über Nacht bei Raumtemperatur. Anderntags wird der Niederschlag abgesaugt und das Filtrat im Hochvakuum eingeengt. Das resultierende Öl wird in 500 ml Essigester gelöst und nacheinander mit je 400 ml Wasser, K₂SO₄/KHSO₄-Lösung, gesättigter NaHCO₃-Lösung und Wasser ausgeschüttelt. Die Essigesterphase wird über Na₂SO₄ getrocknet und eingeengt. Die Substanz wird in 1000 ml Petroläther gelöst, filtriert und über Nacht auf 4°C gekühlt. Anderntags wird die ausgeschiedene Verbindung abfiltriert. Ausbeute 110.5 g, Schmp. 104-105°, [α]_{D}²³ = - 39.9° (c = 1, in Methanol).

### B) H-Ile-Val-OBu^{t} · HCI

105 g Z-Ile-Val-OBu^{t} werden in 1000 ml Methanol gelöst und bei pH 4.5 unter Zugabe von methanolischer Salzsäure und Zusatz von Pd/ BaSO₄ katalytisch hydriert. Nach beendeter Reaktion wird der Katalysator abfiltriert und das Filtrat eingeengt. Das resultierende Öl wird mit 500 ml Ether verrieben und mehrere Tage kühl gestellt. Die Substanz kristallisiert und wird danach abgesaugt. Ausbeute 77.1 g, Schmp. 148-150 °C, [α]_{D}²⁵ = - 22.1° (C = 1, in Wasser).

### C) H-Glu-Ile-Val-OBu^{t}

37 g Z-Glu(OBzl)OH werden wie in Beispiel 21 C) beschrieben mit 32 g H-Ile-Val-OBu^{t} · HCI umgesetzt und aufgearbeitet. Nach katalytischer Hydrierung ist die Verbindung chromatographisch einheitlich.

### D) Lys-Lys-Glu-Ile-Val-OBu^{t}, Acetat

6.8 g Z-Lys(Z)-Lys(Z)-OH werden nach Beispiel 21 D) voraktiviert und mit 4.15 g der wie oben erhaltenen Verbindung umgesetzt. Abspaltung der Schutzgruppen und Reinigung erfolgen analog Beispiel 21 D. Ausbeute 4.9 g. Aminosäureanalyse: Glu 1.00, Val 0.88, lle 0.86, Lys 2.04.

### Beispiel 24

### Lys-Lys-Glu-Ile-Phe-OBu^{t}, Acetat

### A) Z-Ile-Phe-OBu^{t}

Zu einer Lösung von 2.65 g Z-Ile-OH, 2.58 g H-Phe-OBu^{t} und 1.35 g HOBt in 30 ml Dimethylformamid gibt man 1.3 ml N-Ethylmorpholin und bei 0°C 2.2 g DCC. Man lässt 1 Stunde bei 0°C und über Nacht bei Raumtemperatur stehen. Anderntags wird der Niederschlag abgesaugt und das Filtrat eingeengt. Der Rückstand wird zwischen Essigester und Wasser verteilt. Die Essigesterphase wird mit je 20 ml gesättigter NaHCO₃-Lösung, K₂SO₄/KHSO₄-Lösung und NaHCO₃-Lösung ausgeschüttelt, über Na₂SO₄ getrocknet und eingeengt. Das Produkt kristallisiert aus Petroläther. Ausbeute 3.7 g, Schmp. 122-125°.

### B) H-Ile-Phe-OBu^{t} · HCI

3.65 g Z-Ile-Phe-Obu^{t} werden in 100 ml Methanol gelöst und unter Zusatz von Pd/BaSO₄ und Zugabe von 2N methanolischer Salzsäure bei pH 4.5 katalytisch hydriert. Nach beendeter Reaktion wird der Katalysator abgesaugt und das Filtrat eingeengt. Der Rückstand kristallisiert beim Verreiben mit Ether. Ausbeute 2.57 g, Schmp. 107-109°, [α]_{D}²⁴ + 15.9° (c = 1, in Methanol).

### C) H-Glu-Ile-Phe-OBu^{t}

Man setzt analog Beispiel 21 C) 1.85 g Z-Glu(OBzl)-OH, 1.85 g-lle-Phe-OBu^{t} · HCI, und 1.1 g DCC in Anwesenheit von 0.65 ml N-Ethylmorpholin und 0.7 g HOBt in Dimethylformamid um, hydriert katalytisch und isoliert 2.4 g der in Ether unlöslichen Verbindung.

### D) Lys-Lys-Glu-Ile-Phe-OBu^{t}, Acetat

Man aktiviert 3.4 g Z-Lys(Z)-Lys(Z)-OH analog Beispiel 21 D) vor und setzt mit 2.3 g der oben erhaltenen Verbindung um. Die Aufarbeitung erfolgt wie in Beispiel 21 D) beschrieben. Ausbeute 2.5 g. Aminosäureanalyse: Glu 1.00, Ile 0.87, Phe 0.97, Lys 1.98.

### Beispiel 25

### Arg-Lys-Glu-Val-Tyr-OMe, Diacetat

### A) Z-Val-Tyr(Bu^{t})-OMe

Zu einer Lösung von 43.15 g (150 mmol) H-Tyr-(Bu^{t})-OMe · HCI, 37.65 g (150 mmol) Z-Val-OH und 20.25 g (150 mmol) 1-Hydroxybenzotriazol in 250 ml Dimethylformamid gibt man bei 0°C 19.5 ml (ca. 150 mmol) N-Äthylmorpholin und 33 g (160 mmol) DCC zu. Man lässt 1 Stunde bei 0°C rühren und über Nacht bei Raumtemperatur stehen. Vom ausgefallenen Niederschlag wird abgesaugt. Mit wenig Dimethylformamid wird nachgewaschen. Das Filtrat wird in eine Mischung aus 150 ml gesättigter NaHCO₃-Lösung und 3000 ml Wasser unter Rühren eingetragen. Man kühlt auf 4°C ab und saugt den Niederschlag ab. Mit Wasser wird gut gewaschen. Das noch feuchte Produkt wird in 400 ml Essigester gelöst. Von Unlöslichem wird filtriert und Wasser im Scheidetrichter abgetrennt. Die Essigesterphase wird über Na₂SO₄ getrocknet und eingeengt. Der Rückstand wird mit Petroläther verrieben und abgesaugt. Ausbeute 67 g (92%). Schmp. 111-112°, [α]_{D}²¹ - 15.9° (c = 1, Methanol.

### B) H-Val-Tyr(Bu^{t})-OMe · HCI

Zu einer Lösung von 65g (0.134 mol) Z-Val-Tyr-(Bu^{t})-OMe in 300 ml Methanol gibt man Pd/Kohle-Katalysator und leitet unter Rühren und unter Zugabe von ca. 2N methanolischer Salzsäure bei pH 4.5 (Autotitrator) Wasserstoff durch die Lösung, bis keine methanolische Salzsäure mehr aufgenommen wird. Danach wird der Katalysator abgesaugt und das Filtrat eingeengt. Der Rückstand wird mit Äther verrieben, wobei sich die Substanz löst und nach Stehen über Nacht bei 4° kristallin ausfällt. Die Kristalle werden abgesaugt und über P₂O₅ getrocknet. Die Substanz ist hygroskopisch. Ausbeute 44.1 g (85%), Schmp. 88-102°, [α]_{D}²¹ = 31.0° (c = 1, Methanol).

### C) Z-Glu(OBu^{t})-Val-Tyr(Bu^{t})-OMe

Zu einer Lösung von 43.5 g (112.4 mmol) H-Val-Tyr(Bu^{t})-OMe · HCI und 15.1 g (112 mmol) a-Hydroxybenzotriazol in 120 ml Dimethylformamid gibt man bei Raumtemperatur 14.5 ml (113 mmol) N-Äthylmorpholin und 58 g (112 mmol) Z-Glu(O-Bu^{t})-OTcp. Man lässt zwei Stunden bei Raumtemperatur rühren und rührt die Reaktionslösung in eine Mischung von 120 ml gesättigter NaHCO₃-Lösung und 1200 ml Wasser ein. Man kühlt auf 4°C ab und saugt den ausgefallenen Niederschlag ab. Der noch feuchte Niederschlag wird in 1200 ml Essigester gelöst und das anhaftende Wasser im Scheidetrichter entfernt. Man extrahiert noch einmal mit 300 ml Wasser, trocknet über Na₂SO₄ und engt ein. Der Rückstand wird aus Essigester/ Petroläther umgefällt und über Paraffin getrocknet. Ausbeute 70 g (93%), Schmp. 163-164°, [α]_{D}²⁵ = - 24.6° (c = 1, Methanol).

### D) H-Glu(OBu^{t})-Val-Tyr(Bu^{t})-OMe · HCI

70 g (140 mmol) Z-Glu(OBu^{t})-Val-Tyr(Bu^{t})-OMe werden analog Beispiel B katalytisch hydriert. Der Rückstand kristallisiert nicht und wird nach Trocknen im Hochvakuum als amorpher Schaum erhalten. Ausbeute 55 g (92.5%), [α]_{D}²¹ = + 15.3° (c = 1, Methanol).

### E) Z-Lys(Boc)-Glu(OBu^{t})-Val-Tyr(But)-OMe

Zu einer Lösung von 28.6 g (50 mmol) H-Glu(O-Bu^{t})-Val-Tyr(Bu^{t})-OMe und 6.75 g (50 mmol) 1-Hydroxybenzotriazol in 80 ml Dimethylformamid gibt man bei Raumtemperatur 6.5 ml (ca. 51 mmol) N-Äthylmorpholin und 28 g (50 mmol) Z-Lys(Boc)-OTcp. Man rührt zwei Stunden bei Raumtemperatur und trägt die Reaktionslösung in eine Mischung aus 50 ml gesättigter NaHCO₃-Lösung und 600 ml Wasser ein. Man kühlt auf 4 °C ab und saugt den ausgefallenen Niederschlag ab. Der noch feuchte Niederschlag wird in die benötigte Menge Essigester gelöst und das anhaftende Wasser im Scheidetrichter abgetrennt. Die Essigesterphase wird über Na₂SO₄ getrocknet und fast bis zur Trokkene eingeengt. Das Peptid wird aus der konzentrierten Lösung mit Petroläther ausgefällt. Der Niederschlag wird abgesaugt, mit Petroläther gewaschen und über Paraffin getrocknet. Ausbeute 44.3 g (98%), Schmp. 149-152°, [α]_{D}²¹= - 20.4° (c = 1. Methanol).

### F) H-Lys(Boc)-Glu(OBu^{t})-Val-Tyr(Bu^{t})-OMe · HCl

44 g (49 mmol) Z-Lys(Boc)-Glu(OBu^{t})-Val-Tyr-(Bu^{t})-OMe werden analog Beispiel B katalytisch hydriert. Der Rückstand wird mit Äther verrieben. Ausbeute 31.8 g (81 %), Schmp. 175-177°, [α]_{D}²² = - 10.7° (c = 1, Methanol).

### G) Z-Arg(Z₂)-Lys(Boc)-Glu(OBu^{t})-Val-

### Tyr(Bu^{t})-OMe

Zu einer Lösung von 28 g (35 mmol) H-Lys(Boc)-Glu(OBu^{t})-Val-Tyr(But)-OMe · HCI und 4.73 g 1-Hydroxybenzotriazol in 85 ml Dimethylformamid gibt man bei Raumtemperatur 4.55 ml (35.5 mmol) N-Äthylmorpholin und eine Lösung von 26.5 g (35 mmol) Z-Arg(Z₂)-OTcp in 50 ml Dimethylformamid. Man rührt zwei Stunden bei Raumtemperatur und trägt den Ansatz in eine Mischung aus 35 ml gesättigter NaHCO₃-Lösung und 700 ml Wasser ein. Man kühlt auf 4°C ab und saugt den Niederschlag ab und trocknet über P₂O₅. Danach wird die Substanz (44 g) mit 1000 ml Essigester aufgekocht, mit 1000 ml Petroläther versetzt und abgekühlt. Der Niederschlag wird abgesaugt und getrocknet. Ausbeute 43.15 g. Zur weiteren Reinigung wird die Substanz mit 450 ml Methanol aufgekocht. Man lässt auf Raumtemperatur abkühlen und saugt ab, wäscht mit Methanol nach und trocknet im Vakuum. Ausbeute 38.4 (81%), Schmp. 201 °C, [α]_{D}²² = -4.7° (C = 1, Eisessig).

### H) Z-Arg(Z₂)-Lys-Glu-Val-Tyr-OMe

36 g (26.7 mmol) Z-Arg(Z₂)-Lys(Boc)-Glu(OBu^{t})-Val-Tyr(Bu^{t})-OMe werden in 180 ml 90prozentiger Trifluoressigsäure gelöst. Man lässt 1 Stunde bei Raumtemperatur stehen und engt im Vakuum ein. Der Rückstand wird mit Wasser verrieben, abgesaugt und getrocknet. Anschliessend wird die so gewonnene Substanz mit 500 ml Methanol aufgekocht, auf Raumtemperatur abgekühlt, abgesaugt und getrocknet. Ausbeute 18.1 g (61%), Schmp. 196-199° unter Zersetzung, [α]_{D}²¹ = -2:8° (c = 1, Dimethylacetamid). Die Methanol-Mutterlauge wird eingeengt und der Rückstand mit Wasser verrieben, abgesaugt und über P₂O₅ getrocknet. Ausbeute 10.8 g (36.5%), Schmp. 187-191° unter Zersetzung, [α]_{D}²¹ = -3.3° (c = 1, Dimethylacetamid). Gesamtausbeute: 97.5%.

### I) Arg-Lys-Glu-Val-Tyr-OMe, Acetat

17.5 g (15.8 mmol) Z-Arg(Z₂)-Lys-Glu-Val-Tyr-OMe werden in 700 ml 90prozentiger Essigsäure gelöst. Dazu gibt man Pd-Kohle-Katalysator und leitet Wasserstoff durch die Lösung, bis kein COz mehr entweicht. Der Katalysator wird abgesaugt, das Filtrat eingeengt und der Rückstand in Wasser gelöst. Die wässrige Lösung wird, wenn nötig, filtriert und gefriergetrocknet. Ausbeute 12.13 g (_{92%}), [α]_{D}²² = -₃₅._{8°} (c = 1, in Wasser). Aminosäureanalyse: Glu (1.00), Val (0.96), Tyr (0.91), Lys (1.04) Arg (0.95) (Hydrolyse: 24 Stunden bei 120° in 6N HCI).

### Beispiel 26

### Arg-Lys-Glu-Val-Tyr-OH, Acetat

### A) Z-Val-Tyr(Bu^{t})-OBu^{t}

Zu einer Lösung von 33 g (0.1 mol) H-Tyr(Bu^{t})-OBu^{t}, 25.1 g (0.1 mol) Z-Val-OH und 13.5 g 1-Hydroxybenzotriazol in 150 ml Dimethylformamid gibt man bei 0°C 13 ml (ca. 0.1 mol) N-Äthylmorpholin und 22 g (107 mmol) Dicyciohexyicarbodiimid. Man lässt eine Stunde bei 0°C rühren und lässt über Nacht bei Raumtemperatur stehen. Der Niederschlag wird abgesaugt und das Filtrat eingeengt. Der Rückstand wird zwischen 300 ml Essigester und 300 ml Wasser verteilt. Die Essigesterphase wird nacheinander mit je 100 ml gesättigter NaHCO₃-Lösung, KHSO₄/K₂SO₄-Lösung, gesättigter NaHCO₃-Lösung und Wasser ausgeschüttelt. Nach Trocknen über Na₂SO₄ wird eingeengt. Der Rückstand wird mit Petroläther verrieben, gekühlt und abgesaugt. Ausbeute 31.2 g (60%), Schmp. 82-83°, [α]_{D}²¹ = -20.9° (c = 1, Methanol).

### B) H-Val-Tyr(Bu^{t})-OBu^{t} · HCó

Zu einer Lösung von 31 g (58,8 mmol) Z-Val-Tyr(Bu^{t})-OBu^{t} in 300 ml Methanol gibt man Pd/ Kohle-Katalysator und leitet unter Rühren und unter Zugabe von ca. 2N methanolischer Salzsäure bei pH 4.5 (Autotitrator) Wasserstoff durch die Lösung, bis keine methanolische Salzsäure mehr aufgenommen wird. Danach wird der Katalysator abgesaugt und das Filtrat eingeengt. Der Rückstand wird mit Äther verrieben, abgesaugt und getrocknet. Ausbeute 23.6 g (94%), Schmp. 159-161°, [α]_{D}²⁰ = + 19.4° (c = 1, Methanol).

### C) Z-Glu(BOu^{t})-Val-Tyr(Bu^{t})-OBu^{t}

Zu einer Lösung von 8.6 g (20 mmol) H-Val-Tyr-(Bu^{t})-OBu^{t} · HCI und 2.7 g (20 mmol) 1-Hydroxybenzotriazol in 20 ml Dimethylformamid gibt man bei Raumtemperatur 2.6 ml (ca. 20 mmol) N-Äthylmorpholin und 11.35 g (22 mmol) Z-Glu(OBu^{t})-OTcp. Man rührt 2 Stunden bei Raumtemperatur und trägt die Reaktionslösung in eine Mischung aus 30 ml gesättigter NaHCO₃-Lösung und 200 ml Wasser ein. Man kühlt auf 4°C ab und dekantiert das Wasser ab. Der Rückstand wird in 100 ml Essigester gelöst. Die Essigesterphase wird mit 30 ml KHSO₄/K₂SO₄-Lösung und mit 50 ml gesättigter NaHCO₃-Lösung ausgeschüttelt, über Na₂SO₄ getrocknet und eingeengt. Der Rückstand wird in Petroläther gelöst und über Nacht bei 4°C aufbewahrt. Es fällt eine weisse kristalline Substanz aus, die abgesaugt wird. Ausbeute 3.94 g, Schmp. 133°. Die Mutterlauge wird eingeengt und das resultierende Öl (17.6 g) über 200 g Kieselgel chromatographiert. Zuerst wird mit Methylenchlorid eluiert und zum Schluss mit einer Mischung aus Methylenchlorid und Aceton wie 9: 1. Die Fraktionen, die mit im DC mit dem oben beschriebenen Niederschlag identisch sind, werden eingeengt, mit wenig Petroläther verrieben und abgesaugt. Ausbeute 7.74g, Schmp. 133°, [α]_{D}²⁵ = -29.4° (c = 1, Methanol).

### Gesamtausbeute: 11.68 g (82%).

### D) H-Glu(OBu^{t})-Val-Tyr(Bu^{t})-OBu^{t} · HCl

9 g (12.65 mmol) Z-Glu(OBu^{t})-Val-Tyr(Bu^{t})-OBu^{t} werden analog Beispiel B katalytisch hydriert. Der Rückstand kristallisiert nicht und wird nach Trocknen im Hochvakuum als amorpher Schaum erhalten. Ausbeute 7.65 g (98.6%), Schmp. 109-112°, [α]_{D}²⁵ = +2.4° (c = 1, Methanol).

### E) Z-Lys(Boc)-Glu(OBu^{t})-Val-Tyr(Bu^{t})-OBu^{t}

Zu einer Lösung von 3.07 g (5 mmol) H-Glu(O-Bu^{t})-Val-Tyr(Bu^{t})-OBu^{t} · HCI und 675 mg (5 mmol) 1-Hydroxybenzotriazol in 10 ml Dimethylformamid gibt man bei Raumtemperatur 0.65 ml (ca. 5 mmol) N-Äthylmorpholin und eine Lösung von 3.05 g (5.45 mmol) Z-Lys(Boc)-OTcp in 10 ml Dimethylformamid. Man rührt 2 Stunden bei Raumtemperatur und trägt die Reaktionslösung in eine Mischung aus 5 ml gesättigter NaHCO₃-Lösung und 200 ml Wasser ein. Man kühlt auf 4 °C ab und saugt den Niederschlag ab. Der Niederschlag wird über P₂O₅ getrocknet und anschliessend gut mit Petroläther verrieben, abgesaugt und nochmals getrocknet. Ausbeute 4.7 g (100%), Schmp. 104-106°, [α]_{D}²⁴ = -27.5° (c = 1, Methanol).

### F) H-Lys(Boc)-Glu(OBu^{t})-Val-Tyr(Bu^{t})-OBu^{t} HCI

4.6 g (4.9 mmol) Z-Lys(Boc)-Glu(OBu^{t})-Val-Tyr-(Bu^{t})-OBu^{t} werden analog Beispiel B katalytisch hydriert. Der Rückstand wird mit Äther verrieben, abgesaugt und getrocknet. Ausbeute 3.56 g (86%), Schmp. 143-145° unter Zers., [α]_{D} = -19.5° (c = 1, Methanol).

### G) Z-Arg(Zz)-Lys(Boc)-Glu(OBut)-Val-Tyr(Bu^{t})-OBu^{t}

Zu einer Lösung von 1.68 g (2 mmol) H-Lys-(Boc)-Glu(OBut)-Val-Tyr(But)-OBut. HCI und 270 mg (2 mmol) 1-Hydroxybenzotriazol in 5 ml Dimethylformamid gibt man 0.26 ml (2 mmol) N-Äthylmorpholin und eine Lösung von 1.66 g (2.2 mmol) Z-Arg(Z_{z})-OTcp in 2 ml Dimethylformamid. Man lässt zwei Stunden bei Raumtemperatur rühren und gibt anschliessend eine Mischung von 5 ml gesättigter NaHCO₃-Lösung und 50 ml Wasser zu, kühlt auf 4°C ab und saugt den Niederschlag ab, der im Vakuum über P₂O₅ getrocknet wird. Anschliessend verreibt man die Substanz mit einer Mischung 20 ml Essigester und 30 ml Petroläther. Die Substanz wird abgesaugt, mit Petroläther gewaschen und getrocknet. Ausbeute 2.38 g (87%), Schmp. 185-187° unter Zers., [α]_{D} = -5,4° (c = 1, Dimethylacetamid).

### H) H-Arg-Lys(Boc)-Glu(OBu^{t})-Val-Tyr(Bu^{t})-OBu^{t} · 2 HCI

2.2 g (1.61 mmol) Z-Arg(Z_{z})-Lys(Boc)-Glu(OBu^{t})-Val-Tyr(Bu^{t})-OBu^{t} werden analog Beispiel B katalytisch hydriert. Der Rückstand wird mit Äther verrieben und abgesaugt. Ausbeute 1.4 g (84%). [α]_{D} = -17,7° ( c = 1, Methanol).

### I) H-Arg-Lys-Glu-Val-Tyr-OH, Acetat

1 g H-Arg-Lys(Boc)-Glu(OBu^{t})-Val-Tyr(Bu^{t})-OBu^{t} · 2 HCI (0.966 mmol) werden in einer Mischung aus 10 ml Trifluoressigsäure und 1 ml Äthylmercaptan gelöst. Man lässt eine Stunde bei Raumtemperatur stehen und engt ein. Der Rückstand wird erneut mit etwa Methanol gelöst und wieder eingeengt. Der Rückstand wird in Wasser gelöst, von Unlöslichem filtriert und über einen stark basischen Austauscher in Acetatform chromatographiert. Das Eluat wird eingeengt und zur weiteren Reinigung an einem hydroxypropylierten vernetzten Dextrangel in einer Wasser/Essigsäure/n-Butanolmischung chromatographiert. Ausbeute 491 mg (57%), [α]_{D} = -21,8° (c = 1, Wasser).

Aminosäureanalyse: Glu 1.00, Val 0.97, Tyr 0.92, Lys 1.03, Arg 0.99. Proteingehalt: 82%.

### Beispiel 27

### Lys-Arg-Glu-Val-Tyr-OMe, Acetat

### A) Z-Arg(Z₂)-Glu(OBu^{t})-Val-Tyr(Bu^{t})-OMe

Zu einer Lösung von 11.4 g (20 mmol) H-Glu(O-Bu^{t})-Val-Tyr(Bu^{t})-OMe · HCl und 2.7 g (20 mmol) 1-Hydroxybenzotriazol in 40 ml Dimethylformamid gibt man bei Raumtemperatur 2.6 ml (20 mmol) N-Äthylmorpholin und 15.12 g (20 mmol) Z-Arg(Z₂)-OTcp. Man lässt zwei Stunden bei Raumtemperatur rühren. Danach wird der Ansatz mit 400 ml Wasser und 20 ml gesättigter NaHCO₃-Lösung verrührt. Der Niederschlag wird abgesaugt und getrocknet. Aus heissem Essigester wird umkristallisiert. Ausbeute 20 g (91%). Schmp. 186-188°, [a]_{D}²² = -0.9° (c = 1, in Eisessig).

### B) H-Arg-Glu(OBu^{t})-Val-Tyr(Bu^{t})-OMe 2 HCI

18.5 g (16.9 mmol) Z-Arg(Z₂)-Glu(OBu^{t})-Val-Tyr-(Bu^{t})-OMe werden analog Beispiel 26 B katalytisch hydriert und aufgearbeitet. Ausbeute 11.5 g (89%), Schmp. 149-150°, [α]_{D}²² = -9.2° (c = 1, in Methanol).

### C) Z-Lys(Boc)-Arg-Glu(OBu^{t})-Val-Tyr(Bu^{t})-OMe · HCI

Zu einer Lösung von 1.53 g (2 mmol) H-Arg-Glu(OBu^{t})-Val-Tyr(Bu^{t})-OMe · 2 HCI und 0.27 g (2 mmol) 1-Hydroxybenzotriazol in 8 ml Dimethylformamid gibt man bei Raumtemperatur 0.26 ml N-Äthyl-morpholin und 1.12 g (2 mmol) Z-Lys(Boc)-OTcp. Man lässt zwei Stunden bei Raumtemperatur rühren und gibt danach 30 ml Wasser und 2 ml gesättigte NaHCO₃-Lösung zu. Der Niederschlag wird abgesaugt und getrocknet. Zur Reinigung wird zweimal aus Essigester/Petroläther (1 : 1) umgefällt. Ausbeute 1.44 (66%). Schmp. 117-119 unter Zersetzung, [α]_{D}²² = -24.5° (c = 1, in Methanol).

### D) Z-Lys-Arg-Glu-Val-Tyr-OMe, Acetat

1.2 g (1.1 mmol) Z-Lys(Boc)-Arg-Glu(OBu^{t})-Val-Tyr(Bu^{t})-OMe · HCI werden in 10 ml 90prozentiger Trifluoressigsäure gelöst. Man lässt eine Stunde bei Raumtemperatur stehen und engt ein. Der Rückstand wird zwischen 150 ml Wasser und 100 ml Diäthyläther verteilt. Die wässrige Phase wird mit einem schwach basischen Ionenaustauscher in der Acetatform gerührt. Der Austauscher wird abgesaugt und das Filtrat gefriergetrocknet. Ausbeute 880 mg (89%), [α]_{D}²³ = -45.8° (c = 1, in Wasser).

### E) Lys-Arg-Glu-Val-Tyr-OMe, Acetat

800 mg (ca. 0.88 mmol) Z-Lys-Arg-Glu-Val-Tyr-OMe-Acetat werden analog Beispiel 25 I in 90prozentiger Essigsäure katalytisch hydriert und aufgearbeitet. Ausbeute 680 mg (93%), [α]_{D}²² = -31.6° ( c = 1, in Wasser).

### Beispiel 28

### D-Lys-Arg-Glu-Val-Tyr-OMe, Acetat

### A) Z-D-Lys(Boc)-Arg-Glu(OBu^{t})-Val-Tyr(Bu^{t})-OMe · HCI

Zu einer Lösung von 3.06 g (4 mmol) H-Arg-Glu(OBu^{t})-Val-Tyr(Bu^{t})-OMe · 2HCl und 0.54 g (4 mmol) HOBt in 16 ml Dimethylformamid gibt man bei Raumtemperatur 0.54 g (4 mmol) N-Äthylmorpholin und 2.24g (4 mmol) Z-D-Lys(Boc)-OTcp. Man arbeitet analog Beispiel 27 C auf. Ausbeute 4.0 g (92%), Schmp. 167-172°, [α]_{D}²² = -15.5° (c = 1, Methanol).

### B) Z-D-Lys-Arg-Glu-Val-Tyr-OMe, Acetat

3.6 g Z-D-Lys(Boc)-Arg-Glu(OBu^{t})-Val-Tyr(Bu^{t})-0Me · HCl (3.3 mmol) werden in 30 ml 90prozentiger Trifluoressigsäure gelöst und analog Beispiel 27 D aufgearbeitet. Ausbeute 2.81 g (94%), [α]_{D}²³ = -32.4° (c = 1, Wasser).

### C) D-Lys-Arg-Glu-Val-Tyr-OMe, Acetat

2 g (2.2 mmol) Z-D-Lys-Arg-Glu-Val-Tyr-OMe, Acetat werden analog Beispiel 25 I in 90prozentiger Essigsäure katalytisch hydriert und aufgearbeitet. Ausbeute 1.8 g (98%). Zur weiteren Reinigung werden 800 mg an einem hydroxypropylierten vernetzten Dextrangel (ca. 120 g, Säule: 90 x 2.5 cm) in 90prozentigem Methanol chromatographiert. Ausbeute 500 mg.

### Beispiel 29

### Arg-Lys-Glu-Val-Trp-OMe, Acetat

### A) Z-Val-Trp-OMe

Zu einer Lösung von 12.64 g (50 mmol) H-Trp-OMe · HCI, 6,75 g (50 mmol) 1-Hydroxybenzotriazol und 12.55 g (50 mmol) Z-Val-OH in 100 ml Dimethylformamid gibt man bei 0°C 6.5 ml (ca. 50 mmol) N-Äthylmorpholin und 11 g Dicyclohexylcarbodiimid. Man lässt eine Stunde bei 0°C rühren und lässt über Nacht bei Raumtemperatur stehen. Anderntags wird der Niederschlag abgesaugt und das Filtrat mit 50 ml ges. NaHCO₃-Lösung und 1000 ml Wasser verrührt. Man kühlt auf 4°C ab und saugt den Niederschlag ab. Der noch feuchte Niederschlag wird in Essigester gelöst. Die Essigesterphase wird mit Wasser, ges. NaH-CO₃-Lösung, KHSO₄/K₂SO₄-Lösung und nochmals mit ges. NaHCO₃-Lösung ausgeschüttelt, über Na₂SO₄ getrocknet und bis auf einen kleinen Rest eingeengt. Aus der konzentrierten Lösung wird mit Petroläther das Peptid gefällt. Der Niederschlag wird abgesaugt und getrocknet. Ausbeute 19.1 g (84%), Schmp. 146-148°, [α]_{D}²² = -13.9° (c = 1, in Methanol).

### B) H-Val-Trp-OMe · HCl

18.2 g (40.3 mmol) Z-Val-Trp-OMe werden analog Beispiel 25 B katalytisch hydriert. Die Substanz kristallisiert aus Äther. Ausbeute 12.9 g (90%), Schmp. 218-221°, [α]_{D}²² = +14.2° (c = 1, in Methanol).

### C) Z-Glu(OBu^{t})-Val-Trp-OMe

Zu einer Lösung von 12.1 g (34.2 mmol) H-Val-Trp.OMe - HCI und 4.62 g (34.2 mmol) 1-Hydroxybenzotriazol in 50 ml Dimethylformamid gibt man bei Raumtemperatur 4.38 ml (34.2 mmol) N-Äthylmorpholin und 17.7 g (34.2 mmol) Z-Glu(OBu^{t})-OTcp. Man lässt 2 Stunden bei Raumtemperatur rühren und versetzt die Lösung anschliessend mit 35 ml ges. NaHCO₃-Lösung und 350 ml Wasser. Es wird auf 4°C abgekühlt und der Niederschlag abgesaugt. Die feuchte Substanz wird in Essigester gelöst. Die Essigesterphase wird mit 100 ml Wasser extrahiert, über Na₂SO₄ getrocknet und eingeengt. Der Rückstand wird mit Petroläther verrieben und abgesaugt. Ausbeute 20.5 g (94%), Schmp. 158-162°, [α]_{D}²² = -26.1° (c = 1, in Methanol).

### D) H-Glu(OBu^{t})-Val-Trp-OMe · HCI

19.8 g (31 mmol) Z-Glu(OBu^{t})-Val-Trp-OMe werden analog Beispiel 25 B katalytisch hydriert. Die Substanz ist amorph. Ausbeute 15.3 g (91%), [α]_{D}²² = +₉.5° (c = 1, in Methanol).

### E) Z-Lys(Boc)-Glu(OBu^{t})-Val-Trp-OMe

Zu einer Lösung von 14.5 g (26.9 mmol) H-Glu(OBu^{t})-Val-Trp-OMe und 3.63 g (26.9 mmol) 1-Hydroxybenzotriazol in 60 ml Dimethylformamid gibt man 3.45 ml (26.9 mmol) N-Äthylmorpholin und 15.06 g (26.9 mmol) Z-Lys(Boc)-OTcp. Man rührt zwei Stunden bei Raumtemperatur und setzt unter Kühlen 25 ml gesättigte NaHCO₃-Lösung und 300 ml Wasser zu. Der Niederschlag wird abgesaugt und getrocknet. Der Rückstand wird mit Petroläther verrieben und abgesaugt. Zur weiteren Reinigung wird aus Essigester umkristallisiert. Ausbeute 19.3 g, (83%), Schmp. 169-174°, [α]_{D}²² = -28.1° (c = 1, in Methanol).

### F) H-Lys(Boc)-Glu(OBu^{t})-Val-Trp-OMe · HCI

6.3 g (7.28 mmol) Z-Lys(Boc)-Glu(OBu^{t})-Val-Trp-OMe werden analog Beispiel 25 B katalytisch hydriert. Ausbeute 4.8 g (86%), Schmp. 158-162°, [α]_{D}²³ = -16.2° (c = 1, in Methanol).

### G) Z-Arg(Z_{z})-Lys(Boc)-Glu(OBu^{t})-Val-Trp-OMe

Zu einer Lösung von 4.5 g (5.87 mmol) H-Lys-(Boc)-Glu(OBu^{t})-Val-Trp-OMe · HCl und 0.8 g (ca. 5.9 mmol) 1-Hydroxybenzotriazol in 70 ml Dimethylformamid gibt man bei Raumtemperatur 0.75 ml (5.86 mmol) N-Äthylmorpholin und 4.44 g (5.87 mmol) Z-Arg(Z_{z})-OTcp. Man lässt zwei Stunden bei Raumtemperatur rühren und gibt man 5.9 ml gesättigte NaHCO₃₋Lösung und 700 ml Wasser zu. Der Niederschlag wird abgesaugt und getrocknet. Ausbeute 7.4 g. Zur weiteren Reinigung wird die Substanz mit 70 ml Methanol aufgekocht, auf Raumtemperatur abgekühlt und abgesaugt. Ausbeute 6.6 g (87%), Schmp. 189-190°, [α]_{D}²⁰ = -4.7° (c = 1, in Eisessig).

### H) Z-Arg(Zz)-Lys-Glu-Val-Trp-OMe

5 g (3.88 mmol) Z-Arg(Z₂)-Lys(Boc)-Glu(OBu^{t})-Val-Trp-OMe werden zusammen mit 1.2 ml Äthandithiol in 30 ml Trifluoressigsäure gelöst. Nach einer Stunde Stehen bei Raumtemperatur wird die Lösung eingeengt und der Rückstand öfters mit Wasser digeriert, abgesaugt und getrocknet. Anschliessend wird zweimal mit 30 ml Essigester aufgekocht, abgesaugt und getrocknet. Ausbeute 3.97 g (90%). Zur weiteren Reinigung wird die Substanz an Kieselgel in dem Lösungsmittelgemisch Methylenchlorid : Methanol : Wasser : Essigsäure wie 90 :15 : 2 : 2 chromatographiert. Ausbeute 2.78 g (63%). Der amorphe Rückstand wurde ohne Charakterisierung weiterverarbeitet.

### I) H-Arg-Lys-Glu-Val-Trp-OMe, Diacetat

1.4 g Z-Arg(Z₂)-Lys-Glu-Val-OMe (1.23 mmol) werden in 70 ml 90prozentiger wässriger Essigsäure gelöst. Man gibt Palladium-Katalysator zu und leitet Wasserstoff durch die Lösung, bis kein CO_{z} mehr entweicht. Danach wird der Katalysator abgesaugt und das Filtrat eingeengt. Der Rückstand wird in Wasser gelöst, filtriert und gefriergetrocknet. Ausbeute 580 mg (55%). Zur weiteren Reinigung werden 500 mg an einem hydroxypropylierten Dextrangel (100 x 2.5 cm) in 90prozentigem wässrigem Methanol chromatographiert. Ausbeute 215 mg.

### Beispiel 30

### Arg-Lys-Glu-Val-Tyr-O-cyclohexyl, Acetat

### A) Z-Tyr(Bu^{t})-O-cyclohexyl

Zu einer Lösung von 9.27 g (25 mmol) Z-Tyr-(Bu^{t})-OH in 15 ml Pyridin und 30 ml Cyclohexanol gibt man bei 5°C unter Rühren innerhalb von 15 Minuten 20 ml einer 50prozentigen Propylphosphonsäureanhydrid-Lösung in Methylenchlorid zu. Nach beendeter Zugabe lässt man auf Raumtemperatur kommen und lässt über Nacht bei Raumtemperatur stehen. Der Ansatz wird zwischen Diisopropyläther und Wasser verteilt. Die organische Phase wird nacheinander mit KHS0₄-Lösung, NaHCO₃-Lösung und Wasser ausgeschüttelt, über Na₂SO₄ getrocknet und eingeengt. Es bleiben 20.8 g eines farblosen Öls zurück. Zur weiteren Reinigung wird die Substanz über 150 g Kieselgel in einer Methylenchlorid/Aceton-Mischung (9: 1) chromatographiert. Ausbeute 12.2 g farbloses Öl (enthält noch Lösungsmittel).

### B) H-Tyr(Bu^{t})-O-eyclohexyl · HCI

24.5 öliges, methylenchloridhaltiges Z-Tyr(Bu^{t})-O-cyclohexyl (maximal 50 mmol) werden in 350 ml Methanol analog Beispiel 25 B katalytisch hydriert. Der Rückstand wird mit Petroläther verrieben, abgesaugt und getrocknet. Ausbeute 15.9 g (91 %), Schmp. 181° u. Zers., [α]_{D}²² = + 17.8° (c = 1, in Methanol).

### C) Z-Val-Tyr(Bu^{t})-O-cyclohexyl

Zu einer Lösung von 5.03 mg (20 mmol) Z-Val-OH, 7 g (20 mmol) H-Tyr(Bu^{t})-O-cyclohexyl · HCl und 2.7 g (20 mmol) 1-Hydroxybenzotriazol in 20 ml Dimethylformamid gibt man bei 0°C 2.6 ml (ca. 20 mmol) N-Äthylmorpholin und 4.4 g (21.4 mmol) Dicyclohexylcarbodiimid. Man lässt 1 Stunde bei 0°C und anschliessend über Nacht bei Raumtemperatur rühren. Der Niederschlag wird abgesaugt und das Filtrat mit 200 ml Wasser und 20 ml gesättigter NaHCO₃-Lösung versetzt. Der Niederschlag wird abgesaugt und mit Wasser gewaschen. Das noch wasserfeuchte Produkt wird zwischen 150 ml Essigester und 150 ml Wasser verteilt. Die Essigesterphase wird noch einmal mit 100 ml ges. NaH-CO₃-Lösung gewaschen, über Na₂SO₄ getrocknet und eingeengt. Der Rückstand wird mit Petroläther verrieben und abgesaugt. Ausbeute 7.25 g (65%), Schmp. 99-100°, [α]_{D}²³ = -_{23.3°} (c = 1, in Methanol).

### D) H-Val-Tyr(Bu^{t})-O-cyclohexyl · HCI

6.5 g (11.76 mmol) Z-Val-Tyr(Bu^{t})-O-cyclohexyl werden in 150 ml Methanol analog Beispiel 25 B katalytisch hydriert. Der Rückstand wird mit Äther verrieben, gekühlt, abgesaugt und getrocknet. Ausbeute 5.35 g (100%), Schmp. 152-154°, [α]_{D}²² = +20.4° (c = 1, in Methanol).

### E) Z-Glu(OBu^{t})-Val-Tyr(Bu^{t})-O-cyclohexyl

Zu einer Lösung von 4.6 g (10 mmol) H-Val-Tyr-(Bu^{t})-O-cyclohexylhydrochlorid und 1.35 g 1-Hydroxybenzotriazol in 20 ml Dimethylformamid gibt man bei 0° 1.3 ml (10 mmol) N-Äthylmorpholin und 5.2 g (10 mmol) Z-Glu(OBu^{t})-OTcp. Man rührt zwei Stunden bei Raumtemperatur und versetzt den Ansatz anschliessend unter Rühren mit einer Mischung von 200 ml Wasser und 20 ml gesättigter NaHCO₃-Lösung. Der Niederschlag wird abgesaugt und mit Wasser gewaschen. Der noch feuchte Niederschlag wird in 200 ml Essigester gelöst. Das anhaftende Wasser wird abgetrennt. Die Essigesterphase wird noch einmal mit 50 ml Wasser ausgeschüttelt, über Na₂SO₄ getrocknet und eingeengt. Aus Essigester/Petroläther wird umgefällt. Der Niederschlag wird abgesaugt, mit Petroläther gewaschen und getrocknet. Ausbeute 5.4 g (73%), Schmp. 160-162°, [α]_{D}²⁴ = -28.3° (c = 1, in Methanol).

### F) H-Glu(OBu^{t})-Val-Tyr(Bu^{t})-O-cyclohexyl · HCI

4.5 g (6.1 mmol) Z-Glu(OBu^{t})-Val-Tyr(Bu^{t})-O-cyclohexyl werden in 250 ml Methanol analog Beispiel 25 B katalytisch hydriert. Es bleiben 3.2 g (82%) eines amorphen Schaums zurück, der ohne Charakterisierung weiterverarbeitet wird.

### G) Z-Lys(Boc)-Glu(OBu^{t})-Val-Tyr(Bu^{t})-O-cyclohexyl

Zu einer Lösung von 3.2 g (5 mmol) H-Glu(O-Bu^{t})-Val-Tyr(Bu^{t})-O-cyclohexyl · HCI und 675 mg (5 mmol) 1-Hydroxybenzotriazol in 10 ml Dimethylformamid gibt man bei Raumtemperatur 0.65 ml (5 mmol) N-Äthylmorpholin und 2.8 g (5 mmol) Z-Lys-(Boc)-OTcp. Man rührt zwei Stunden bei Raumtemperatur und arbeitet analog E auf. Ausbeute 3.5 g (72%), Schmp. 141-145°, [α]_{D}²⁴= -28.0° (c = 1, in Methanol).

### H) H-Lys(Boc)-Glu(OBu^{t})-Val-Tyr(Bu^{t})-O-cyclohexyl · HCI

3 g (3.1 mmol) Z-Lys(Boc)-Glu(OBu^{t})-Val-Tyr-(Bu^{t})-O-cyclohexyl werden in 150 ml Methanol analog Beispiel 25 B katalytisch hydriert. Der Rückstand wird mit Äther verrieben, abgesaugt und getrocknet. Ausbeute 2.47 g (92%), Schmp. 184-186°, [α]_{D}²⁴ = -15.4° (c = 1, in Methanol).

### I) Z-Arg(Z₂)-Lys(Boc)-Glu(OBu^{t})-Val-Tyr-(Bu^{t})-O-cyclohexyl

Zu einer Lösung von 2.0 g (2.3 mmol) H-Lys-(Boc)-Glu(OBu^{t})-Val-Tyr(Bu^{t})-O-cyclohexyl · HCI und 310 mg 1-Hydroxybenzotriazol (2.3 mmol) in 8 ml Dimethylformamid gibt man bei Raumtemperatur 0.3 ml (2.3 mmol) N-Äthylmorpholin und eine Lösung von 1.74 g (2.3 mmol) Z-Arg(Z₂)-OTcp in 3 ml Dimethylformamid. Man lässt anschliessend zwei Stunden bei Raumtemperatur rühren und versetzt den Ansatz mit 3 ml gesättigter NaH-CO₃-Lösung und 50 ml Wasser. Der Niederschlag wird abgesaugt und getrocknet. Zur Reinigung wird die Substanz in heissem Essigester gelöst und mit Petroläther gefällt. Der Niederschlag wird abgesaugt, mit Petroläther gewaschen und getrocknet. Ausbeute 3 g (94%), Schmp. 184-185°, [α]_{D}²⁴ = -_{9.2}° (c = 1, in Eisessig).

K) Z-Arg(Z₂)-Lys-Glu-Val-Tyr-O-cyclohexyl 2.5 g (1.8 mmol) Z-Arg(Z₂)-Lys(Boc)-Glu(OBu^{t})-Val-Tyr(Bu^{t})-O-cyclohexyl werden in 12.5 ml 90prozentiger Trifluoressigsäure gelöst. Man lässt eine Stunde bei Raumtemperatur stehen und engt i. V. ein. Der Rückstand wird mit Wasser verrieben, abgesaugt und getrocknet. Ausbeute 2.5 g (die Substanz enthält noch Trifluoressigsäure und Wasser). Schmp. 167-171° [α]_{D}²⁶ = -15.7° (c = 1, Eisessig).

### L) Arg-Lys-Glu-Val-Tyr-O-cyclohexyl, Acetat

2 g des nach Beispiel K gewonnenen Z-Arg(Z_{z})-Lys-Glu-Val-Tyr-O-cyclohexyl (maximal 1.44 mmol) werden in 150 ml 90prozentiger Essigsäure analog Beispiel 25 1 katalytisch hydriert. Der Rückstand wird in Wasser über einen schwach basischen Ionenaustauscher in Acetatform (30 x 2 cm) chromatographiert. Das peptidhaltige Eluat wird gefriergetrocknet. Ausbeute 1.18g (91%). Zur weiteren Reinigung werden 800 mg an einem hydroxypropylierten vernetzten Dextrangel in 90prozentigem Methanol chromatographiert.

Ausbeute 600 mg, [α]_{D}²⁴ = -48° (c = 1, Wasser).

Aminosäureanalyse: Glu (1.00), Val (1.02), Tyr (0.91), Lys (0.99), Arg (0.99), Gehalt an Peptidbase: 84%.

### Beispiel 31

### Arg-Lys-Glu-lle-Tyr-O-cyclohexyl, Acetat

### A) Z-Ile-Tyr(Bu^{t})-O-cyclohexyl

Analog Beispiel 30 C werden 5.8 g (20 mmol) Z-lle-OH und 7 g (20 mmol) H-Tyr(Bu^{t})-0-cyclohexyl · HCI kondensiert. Ausbeute 7.7 g (68%), Schmp. 114-115°, [α]_{D}²³= -24.8° (c = 1, Methanol).

### B) H-Ile-Tyr(Bu^{t})-O-cyclohexyl · HCI

Analog Beispiel 30 D werden 7 g (15.4 mmol) Z-Ile-Tyr(Bu^{t})-O-cyclohexyl katalytisch in 150 ml Methanol hydriert. Ausbeute 5.7 g (79%), Schmp. 136-138°, [α]_{D}²² +19.5° (c = 1, Methanol).

### C) Z-Glu(OBu^{t})-Ile-Tyr(Bu^{t})-O-cyclohexyl

Analog Beispiel 30 E werden 4.7 g (10 mmol) H-Ile-Tyr(Bu^{t})-O-cyclohexyl · HCI und 5.2 g Z-Glu(O-Bu^{t})-OTcp (10 mmol) kondensiert Ausbeute 5.9 g (79%), Schmp. 164-165°, [α]_{D}²⁴ = -28.1° (c = 1, Methanol).

### D) Z-Lys(Boc)-Glu(OBu^{t})-Ile-Tyr(Bu^{t})-O-cyclohexyl

Analog Beispiel 30 F werden 5 g (6.65 mmol) Z-Glu(OBu^{t})-Ile-Tyr(Bu^{t})-O-cyclohexyl katalytisch in 200 ml Methanol hydriert. Ausbeute 4.3 g (99%).

Die oben gewonnenen 4.3 g (6.6 mmol) H-Glu(O-Bu^{t})-Ile-Tyr(Bu^{t})-O-cyclohexyl · HCI werden analog Beispiel 30 G mit 3.7 g (6.6 mmol) Z-Lys(Boc)-OTcp umgesetzt. Ausbeute 4.4 g (68%), Schmp. 148-151°, [α]_{D}²⁴ = -27.8° (c = 1, in Methanol).

### E) H-Lys(Boc)-Glu(OBu^{t})-Ile-Tyr(Bu^{t})-O-cyciohexy) · HCI

Analog Beispiel 30 H werden 4.0 g (4.08 mmol) Z-Lys(Boc)Glu(OBu^{t})-Ile-Tyr(Bu^{t})-O-cyclohexyl katalytisch hydriert. Ausbeute 3.26 g (90%), Schmp. 179-180°, [α]_{D}²⁴ = -17.3° (c = 1, in Methanol).

### F) Z-Arg(Z₂)-Lys(Boc)-Gtu(OBu^{t})-Ile-Tyr-(Bu^{t})-O-cyclohexyl

Analog Beispiel 30 I werden 2.5 g (2.83 mmol) H-Lys(Boc)-Glu(OBu^{t})-Ile-Tyr(Bu^{t})-O-cyclohexyl · HCl mit 2.2 g Z-Arg(Z₂)-OTcp (2.9 mmol) umgesetzt.

Ausbeute 3.9 g (98%), Schmp. 182-184°, [α]_{D}²⁴ = - 9.5° ( c = 1, in Eisessig).

### G) Z-Arg(Z₂)-Lys-Glu-Ile-Tyr-O-cyclohexyl

Analog Beispiel 30 K werden 3.5 g (2.5 mmol) Z-Arg(Z₂)-Lys(Boc)-Glu(OBu^{t})-Ile-Tyr(Bu^{t})-O-cyclohexyl mit 90prozentiger Trifluoressigsäure behandelt. Ausbeute 3.4 g (die Substanz enthält noch Trifluoressigsäure und Wasser), Schmp. 176-179 u. Zers., [α]_{D}²⁶ = -16.6° (c = 1, in Eisessig).

### H) H-Arg-Lys-Glu-Ile-Tyr-O-cyclohexyl, Acetat

Analog Beispiel 30 L werden 3 g (ca. 2.2 mmol) Z-Arg(Z₂)-Lys-Glu-Ile-Tyr-O-cyclohexyl in 150 ml 90prozentiger Essigsäure katalytisch hydriert und aufgearbeitet. Ausbeute 1.675 g (76%). Zur weiteren Reinigung werden 800 mg an einem hydroxypropylierten vernetzten Dextrangel in 90prozentigem Methanol chromatographiert. Ausbeute 560 mg, [α]_{D}²⁵ = -46.1° (c = 1, Wasser).

### Beispiel 32

### Arg-Lys-Glu-Val-Tyr-O-n-hexyl, Acetat

### A) Z-Tyr(Bu^{t})-O-n-hexyl

Zu einer Lösung von 18.6 g (50 mmol) Z-Tyr-(Bu^{t})-OH in 30 ml Pyridin und 30 ml n-Hexanol gibt man bei 5°C unter Rühren innerhalb von 15 Minuten 40 ml einer 50prozentigen Propylphosphonsäureanhydrid-Lösung in Methylenchlorid zu. Man arbeitet dann analog Beispiel 30 A. Ausbeute 19.2 g eines farblosen Öls (85%).

### B) H-Tyr(Bu^{t})-O-n-hexyl · HCI

19.2 g (42 mmol) öliges Z-Tyr(Bu^{t})-O-n-hexyl werden in 300 ml Methanol analog Beispiel 25 B katalytisch hydriert. Die Substanz kristallisiert aus Diisopropyläther. Ausbeute 10.11 g (66%). Schmp. 95-96°, [α] 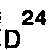 = +11.7° (c = 1, Methanol).

### C) Z-Val-Tyr(Bu^{t})-O-n-hexyl

Analog Beispiel 30 C werden 5.37 g (15 mmol) H-Tyr(Bu^{t})-O-n-hexyl-hydrochlorid und 3.77 g (15 mmol) Z-Val-OH kondensiert. Ausbeute 6.83 g (82%), Schmp. 99°, [α]_{D}²³ = -20.3° (c = 1, Methanol).

### D) H-Val-Tyr(Bu^{t})-O-n-hexyl . HCI

Analog Beispiel 30 D werden 6.5 g Z-Val-Tyr-(Bu^{t})-O-n-hexyl (11.7 mmol) katalytisch hydriert. Kristallisiert aus Äther. Ausbeute 5.28 (99%), Schmp. 138-139°, [α]_{D}²³ = + 22.4° (c = 1, Methanol).

### E) Z-Glu(OBu^{t})-Val-Tyr(Bu^{t})-O-n-hexyl

Analog Beispiel 30 E werden 5.02 g (11 mmol) H-Val-Tyr(Bu^{t})-O-n-hexyl · HCI und 5.93 g (11.5 mmol) Z-Glu(OBu^{t})-OTcp kondensiert. Ausbeute 5.65 g (69%), Schmp. 131°, [α]_{D}²⁶ = -24.6° (c = 1, Methanol).

### F) H-Glu(OBu^{t})-Val-Tyr(Bu^{t})-O-n-hexyl · HCl

Analog Beispiel 30 F werden 5.2 g (7.03 mmol) Z-Glu(OBu^{t})-Val-Tyr(Bu^{t})-O-n-hexyl katalytisch hydriert. Der Rückstand wird scharf getrocknet. Er ist in Äther löslich. Ausbeute 3.9 g (86%), Schmp. 105-110°, [α]_{D}²⁶ = +7.3° (c = 1, in Methanol).

### G) Z-Lys(Boc)-Glu(OBu^{t})-Val-Tyr(Bu^{t})-O-n-hexyl

Analog Beispiel 30 G werden 3.21 g (5 mmol) H-Glu(OBu^{t})-Val-Tyr(Bu^{t})-O-n-hexyl · HCI und 3.07 g (5.5 mmol) Z-Lys(Boc)-OTCp kondensiert. Die Substanz wird mit Äther ausgekocht und warm abgesaugt. Ausbeute 3.87 g (80%), Schmp. 161-162°, [α]_{D}²⁵ = -28.4° (c = 1, Methanol).

### H) H-Lys(Boc)-Glu(OBu^{t})-Val-Tyr(Bu^{t})-O-n-hexyl

Analog Beispiel 30 H werden 3.4 g (3.5 mmol) Z-Lys(Boc)-Glu(OBu^{t})-Val-Tyr(Bu^{t})-O-n-hexyl katalytisch hydriert. Rückstand wird mit Äther verrieben. Ausbeute 2.68 g (88%), Schmp. 185-188°, [α]_{D}²⁴ = -13.3° (c = 1, Methanol).

### I) Z-Arg(Z₂)-Lys(Boc)-Glu(OBu^{t})-Val-Tyr(Bu^{t})· O-n-hexyl

Analog Beispiel 30 I werden 2.52 g (2.9 mmol) H-Lys(Boc)-Glu(OBu^{t})-Val-Tyr(Bu^{t})-O-n-hexyl · HCI mit 2.27 g (3 mmol) Z-Arg(Z₂)-OTcp umgesetzt. Die Substanz wurde mit 50 ml Alkohol zum Sieden erhitzt und heiss abgesaugt. Ausbeute 3.43 g (85%), Schmp. 190°, [α]_{D}²³ = -6.7° (c = 1, Dimethylformamid).

### K) Z-Arg(Z₂)-Lys-Glu-Val-Tyr-O-n-hexyl

Analog Beispiel 30 K werden 3.1 g (ca. 2.2 mmol) Z-Arg(Z₂)-Lys(Boc)-Glu(OBu^{t})-Val-Tyr-(Bu^{t})-O-n-hexyl mit 90prozentiger Trifluoressigsäure behandelt. Ausbeute 2.42 g (93%).

### L) H-Arg-Lys-Glu-Val-Tyr-O-n-hexyl, Acetat

Analog Beispiel 30 L werden 2.42 g (1.73 mmol) Z-Arg(Z₂)-Lys-Giu-Val-Tyr-O-n-hexyl in 90prozentiger Essigsäure katalytisch hydriert und aufgearbeitet. Ausbeute 1.32 g. Zur weiteren Reinigung werden 800 mg an einem hydroxypropylierten vernetzten Dextrangel in 90%igem Methanol chromatographiert. Ausbeute 531.3 mg, [α]_{D}²² = -43.5° (c = 1, Wasser).

### Beispiel 33

### Arg-Lys-Glu-Val-Tyr-O-n-butyl, Acetat

### A) Z-Tyr(Bu^{t})-O-n-butyl

Zu einer Lösung von 18.6 g (50 mmol) Z-Tyr(-Bu^{t})-OH in 30 ml Pyridin und 30 ml n-Butanol gibt man bei 5°C unter Rühren innerhalb von 15 Minuten 40 ml einer 50prozentigen Propylphosphonsäureanhydrid-Lösung in Methylenchlorid zu. Man arbeitet analog Beispiel 30 A auf. Ausbeute 16.6 g farbloses Öl (78%).

### B) H-Tyr(Bu^{t})-O-n-butyl · HCI

11 g (25.7 mmol) öliges Z-Tyr(Bu^{t})-O-n-butyl werden analog Beispiel 25 B katalytisch hydriert. Kristallisiert aus Äther. Ausbeute 6.36 g (72%), Schmp. 134°, [α]_{D}²² = +12.4° (c = 1, Methanol).

### C) Z-Val-Tyr(Bu^{t})-O-n-butyl

Analog Beispiel 30 C werden 6.6 g (20 mmol) H-Tyr(Bu^{t})-O-n-butyl-hydrochlorid und 5.01 g (20 mmol) Z-Val-OH kondensiert. Ausbeute 9.73 g (92%). Zur Reinigung wird die Substanz in Methylenchlorid/Aceton wie 9: 1 an einer Kieselgelsäure (100 g) chromatographiert. Ausbeute 7.7 g (73%), Schmp. 91°.

### D) H-Val-Tyr(Bu^{t})-O-n-butyl · HCI

Analog Beispiel 30 D werden 6.87 g (ca. 13 mmol) Z-Val-Tyr(Bu^{t})-O-n-butyl katalytisch hydriert. Kristallisiert aus Äther. Ausbeute 5.31 g (95%), Schmp. 113-115° u. Zers., [α]_{D}²⁵ = +4.0° (c = 1, Methanol).

### E) Z-Glu(OBu^{t})-Val-Tyr(Bu^{t})-O-n-butyl

Analog Beispiel 30 E werden 5.15 g (12 mmol) H-Val-Tyr(Bu^{t})-O-n-butyl · HCI mit 6.45 g (12.5 mmol) Z-Glu(OBu^{t})-OTcp umgesetzt. Ausbeute 7.31 g (85%), Schmp. 155°, [a]_{D}²⁵ = -25.5° (c = 1, Methanol).

### F) H-Glu(OBu^{t})-Val-Tyr(Bu^{t})-O-n-butyl · HCI

Analog Beispiel 30 F werden 7.12 g (10 mmol) Z-Glu(OBu^{t})-Val-Tyr(Bu^{t})-O-n-butyl katalytisch hydriert. Der Rückstand wird scharf getrocknet. Er ist in Äther löslich. Ausbeute 5.96 g (97%), Schmp. 110°, [α]_{D}²³ = +7.4° (c = 1, Methanol).

### G) Z-Lys(Boc)-Glu(OBu^{t})-Val-Tyr(Bu^{t})-O-n-butyl

Analog Beispiel 30 G werden 4.91 g (8 mmol) H-Glu(OBu^{t})-Val-Tyr(Bu^{t})-O-n-butyl · HCl mit 5.03 g (9 mmol) Z-Lys(Boc)-OTcp umgesetzt. Ausbeute 6.18 g (82%), Schmp. 146°, [a]_{D}²² = -_{28.6°} (c = 1, Methanol).

### H) H-Lys(Boc)-Glu(OBu^{t})-Val-Tyr(Bu^{t})-O-n-butyl . HCI

Analog Beispiel 30 H werden 5.65 g (6 mmol) Z-Lys(Boc)-Glu(OBu^{t})-Val-Tyr(Bu^{t})-O-n-butyl katalytisch hydriert. Rückstand wird mit Äther verrieben. Ausbeute 6.3 g (95%), Schmp. 179-180° u. Zers. [α]_{D}²² = -16.4° (c = 1, Methanol).

### I) Z-Arg(Z₂)-Lys(Boc)-Glu(OBu^{t})-Val-Tyr(Bu^{t})-O-n-butyl

Analog Beispiel 30 I werden 4.21 g (5 mmol) H-Lys(Boc)-Glu(OBu^{t})-Val-Tyr(Bu^{t})-O-n-butyl · HCI mit 4.16 g (5.5 mmol) Z-Arg(Z₂)-OTcp umgesetzt. Die Substanz wurde mit 75 ml Alkohol zum Sieden erhitzt und warm abgesaugt. Ausbeute 6.1 g (89%), Schmp. 191°, [α]_{D}²² = -6.3° (c = 1, Dimethylformamid).

### K) H-Arg-Lys-Glu-Val-Tyr-O-n-butyl, Acetat

Analog Beispiel 30 K werden 5.74 g (4.2 mmol) Z-Arg(Z₂)-Lys(Boc)-Glu(OBu^{t})-Val-Tyr(Bu^{t})-O-n-butyl mit 90 prozentiger Trifluoressigsäure behandelt. Ausbeute 4.99 g. 4.9 g der oben gewonnenen Substanz werden analog Beispiel 30 L katalytisch hydriert und aufgearbeitet. Ausbeute 3.151 g. Zur weiteren Reinigung werden 800 mg Substanz an einem hydroxypropylierten vernetzten Dextrangel in 90prozentigem Methanol chromatographiert. Ausbeute 604 mg. [α]_{D}²³ = -46.6° (c = 1, Wasser).

### Beispiel 34

### Arg-Lys-Glu-Val-Tyr-O-CH(CH₃)₂, Acetat

### A) H-Tyr(Bu^{t})-O-CH(CH₃)₂ · HCl

Analog Beispiel 30 A werden 18.6 g (50 mmol) Z-Tyr(Bu^{t})-OH in 30 ml Pyridin mit 30 ml Isopropanol und 40 ml einer 50prozentigen Propylphosphonsäureanhydrid-Lösung in Methylenchlorid umgesetzt. Das resultierende Öl wird analog Beispiel 25 K katalytisch hydriert. Die Substanz kristallisiert aus Äther, Ausbeute 13.15g g (83%), Schmp. 161-163°, [α]_{D}²³ = +15.7° (c = 1, Methanol).

### B) Z-Val-Tyr(Bu^{t})-O-CH(CH₃)₂

Analog Beispiel 30 C werden 7.9 g (25 mmol) H-Tyr(Bu^{t})-O-CH(CH₃)₂-Hydrochlorid und 6.27 g (25 mmol) Z-Val-OH kondensiert. Ausbeute 11.8g g (92%), Schmp. 133-134°, [α]_{D}²⁶ = -25.2° (c = 1, Methanol).

### C) H-Val-Tyr(Bu^{t})-O-CH(CH₃)₂ · HCl

Analog Beispiel 30 D werden 11.26 g (22 mmol) Z-Val-Tyr(Bu^{t})-O-CH(CH₃)₂ katalytisch hydriert. Ausbeute 8.88 g (97%), Schmp. 126° u. Zers., [a]o25 = +20.8° (c = 1, Methanol).

### D) Z-Gfu(OBu^{t})-Val-Tyr(Bu^{t})-O-CH(CH₃)₂

Analog Beispiel 30 E werden 8.3 g (20 mmol) H-Val-Tyr(Bu^{t})-O-CH(CH₃)₂ · HCI mit 10.83 g (21 mmol) Z-Glu(OBu^{t})-OTcp umgesetzt. Ausbeute 7.42 g (53%), Schmp. 159°, [a]ₒ²⁵ = -27.6° (c = 1, Methanol).

### E) H-Glu(OBu^{t})-Val-Tyr(Bu^{t})-O-CH(CH₃)₂ · HCI

Analog Beispiel 30 F werden 6.96 g (10 mmol) Z-Glu(OBu^{t})-Val-Tyr(Bu^{t})-O-CH(CH₃)₂ katalytisch hydriert. Der Rückstand wird scharf getrocknet. Er ist in Äther löslich. Ausbeute 5.82 g (97%), Schmp. 133°, [α]_{D}²³ = +6.5° (c = 1, Methanol).

### F) Z-Lys(Boc)-Glu(OBu^{t})-Val-Tyr(Bu^{t})-O-CH(CH₃)₂

Analog Beispiel 30 G werden 5.68 g (9.5 mmol) H-Glu(OBu^{t})-Val-Tyr(Bu^{t})-O-CH(CH₃)₂ · HCI mit 5.87 g (10.5 mmol) Z-Lys(Boc)-OTcp umgesetzt. Ausbeute 8.0 g (91%), Schmp. 14₉°, [α]_{D}²² = -30.2° (c = 1, Methanol).

### G) H-Lys(Boc)-Glu(OBu^{t})-Val-Tyr(Bu^{t})-O-CH(CH₃)₂ · HCI

Analog Beispiel 30 H werden 7.4 g (8 mmol) Z-Lys(Boc)-Glu(OBu^{t})-Val-Tyr(Bu^{t})-O-n-butyl katalytisch hydriert. Rückstand wird mit Äther verrieben. Ausbeute 6.3 g (95%), Schmp. 179-180° u. Zers. [α]_{D}²² = -16.4° (c = 1, Methanol).

### H) Z-Arg(Z₂)-Lys(Boc)-Glu(OBu^{t})-Val-Tyr(Bu^{t})-O-CH(CH₃)₂

Analog Beispiel 30 I werden 5.79 g (7 mmol) H-Lys(Boc)-Glu(OBu^{t})-Val-Tyr(Bu^{t})-O-CH(CH₃)₂ HCI mit 5.67 g Z-Arg(Z₂)-OTcp umgesetzt. Die Substanz wurde mit 100 ml Alkohol zum Sieden erhitzt und warm abgesaugt. Ausbeute 7.23 g (76.5%), Schmp. 188°, [α)_{D}²² = -7.3° (c = 1, in Dimethylformamid).

### I) H-Arg-Lys-Glu-Val-Tyr-O-CH(CH₃)_{z}, Acetat

Analog Beispiel 30 K werden 6.75 g (5 mmol) Z-Arg(Z₂)-Lys(Boc)-Glu(OBu^{t})-Val-Tyr(Bu^{t})-O-CH(CH₃)₂ mit 90prozentigerTrifluoressigsäure behandelt. Ausbeute 5.66 g. 5.6 g der oben gewonnenen Substanz werden analog Beispiel 30 L katalytisch hydriert und aufgearbeitet. Ausbeute 3.449 g. Zur weiteren Reinigung werden 800 mg der Substanz an einem hydroxypropylierten vernetzten Dextrangel in 90prozentigem Methanol chromatographiert. Ausbeute 642 mg. [α]_{D}²³ = -42.0° (c = 1, Wasser).

### Beispiel 35

### Arg-Lys-Glu-Val-Tyr-NH-C₂H₅, Acetat

### A) Z-Tyr(Bu^{t})-NH-C₂H₅

Zu einer Lösung von 9.27 g (25 mmol) Z-Tyr-(Bu^{t})-OH, 2.03 g (25 mmol) Äthylaminhydrochlorid und 3.38 g 1-Hydroxybenzotriazol in 50 ml Dimethylformamid gibt man bei 0°C 3.2 ml (25 mmol) N-Äthylmorpholin und 5.56 g (27 mmol) Dicyclohexylcarbodiimid. Man lässt zwei Stunden bei 0°C und 5 Stunden bei Raumtemperatur rühren, lässt über Nacht bei Raumtemperatur stehen. Der Niederschlag wird abgesaugt und das Filtrat eingeengt. Das resultierende Öl wird zwischen Essigester und Wasser verteilt. Die Essigesterphase wird mit KHSO₄-Lösung, NaHCO₃-Lösung und Wasser ausgeschüttelt, über Na₂SO₄ getrockrel und eingeengt. Der Rückstand kristallisiert aus Petroläther. Ausbeute 9.63 g (96%). Schmp. 107-110°, [α]_{D}²² = -0.5° (c = 1, Methanol).

### B) H-Tyr(Bu^{t})-NH-C₂H₅ · HCI

9.5 g (23.8 mmol) Z-Tyr(Bu^{t})-NH-C₂H₅ werden analog Beispiel 25 B katalytisch hydriert. Kristallisiert aus Äther. Ausbeute 6.9 g (96%), Schmp. 163° u. Zers., [α]_{D}²⁴ = +45.2° (c = 1, in Methanol).

### C) Z-Val-Tyr(Bu^{t})-NH-C₂H₅

Analog Beispiel 30 C werden 6.27 g (25 mmol) Z-Val-OH und 7.52 g (25 mmol) H-Tyr(Bu^{t})-NH-C₂H₅ · HCI kondensiert. Die mit Wasser ausgefällte Substanz wird in 70 ml 95prozentigem Alkohol gelöst, heiss filtriert und gekühlt (3 Stunden bei 4°C). Der Niederschlag wird abgesaugt. Ausbeute 9.01 g (72.5%), Schmp. 181°, [α]_{D}²³ = -39.8° (c = 1, Dimethylformamid).

### D) H-Val-Tyr(Bu^{t})-NH-C₂H₅ · HCI

8.7 g (17.5 mmol) Z-Val-Tyr(Bu^{t})-NH-C₂H₅ werden in 125 ml Methanol und 50 ml Dimethylformamid analog Beispiel 25 B katalytisch hydriert. Die Substanz kristallisiert aus Äther. Ausbeute 6.52 g (93%), Schmp. 203-205° u. Zers., [α]_{D}²³ = +₂₈,₀° (c = 1, Methanol).

### E) Z-Glu(OBu^{t})-Val-Tyr(Bu^{t})-NH-C₂H₅

6.18 g (15.5 mmol) H-Val-Tyr(Bu^{t})-NH-C₂H₅ · HCI werden analog Beispiel 30 E mit 8.25 g (16 mmol) Z-Glu(OBu^{t})-OTcp umgesetzt. Die mit Wasser ausgefällte Substanz wird in 120 ml 95prozentigem Alkohol heiss gelöst und heiss filtriert. Das Filtrat lässt man über Nacht bei 4°C stehen und saugt den Niederschlag ab. Ausbeute 8.12 g (76%), Schmp. 209°, [α]_{D}²⁴ = -24.4° (c = 1, Dimethylformamid).

### F) H-Glu(OBu^{t})-Val-Tyr(Bu^{t})-NH-C₂H₅ · HCI

7.51 g (11 mmol) Z-Glu(OBu^{t})-Val-Tyr(Bu^{t})-NH-C₂H₅ werden analog Beispiel 35 D katalytisch hydriert. Die Substanz kristallisiert aus Äther. Ausbeute 6.35 g (98%), Schmp. 199-200° unter Zers., [α]_{D}²³ = -6.7° (c = 1, Methanol).

### G) Z-Lys(Boc)-Glu(OBu^{t})-Val-Tyr(Bu^{t})-NH-C₂H₅

Analog Beispiel 30 G werden 5.85 g (10 mmol) H-Glu(OBu^{t})-Val-Tyr(Bu^{t})-NH-C₂H₅ · HCI mit 5.87 g (10.5 mmol) Z-Lys(Boc)-OTcp umgesetzt. Die mit Wasser ausgefällte Substanz wird aus 100 ml 94prozentigem Alkohol umkristallisiert. Von Unlöslichem wird heiss abgesaugt. Man lässt über Nacht bei 4°C stehen und saugt dann den Niederschlag ab. Ausbeute 6.37 g (67%), Schmp. 216-217°, [α]_{D}²² = -26.8° (c = 1, in Dimethylformamid).

### H) H-Lys(Boc)-Glu(OBu^{t})-Val-Tyr(Bu^{t})-NH-C₂H₅ · HCI

6 g (6.66 mmol) Z-Lys(Boc)-Glu(OBu^{t})-Val-Tyr-(Bu^{t})-NH-C₂H₅ werden analog Beispiel 25 B katalytisch hydriert. Die Substanz kristallisiert aus Äther. Ausbeute 4.77 g (89%), Schmp. 240-242° unter Zers., [α]_{D}²⁶ = -24.9° (c = 1, Methanol).

### I) Z-Arg(Z₂)-Lys(Boc)-Glu(OBu^{t})-Val-Tyr(Bu^{t})-NH-C₂H₅

Analog Beispiel 30 I werden 4.07 g (5 mmol) H-Lys(Boc)-Glu(OBu^{t})-Val-Tyr(Bu^{t})-NH-C₂N₅ · HCl mit 3.97 g (5.25 mmol) Z-Arg(Z₂)-OTcp umgesetzt. Die mit Wasser ausgefällte Substanz wird getrocknet und mit Äther verrieben. Ausbeute 6.66 g (99.8%), Schmp. 222-224° unter Zers. [α]_{D}²⁵ = (c = 1, Essigsäure).

### K) H-Arg-Lys-Glu-Val-Tyr-NH-C₂H₅, Acetat

Analog Beispiel 30 K werden'6.0 g (4.5 mmol) Z-Arg(Z₂)-Lys(Boc)-Glu(OBu^{t})-Val-Tyr(Bu^{t})-NH-C₂H₅ mit 90prozentiger Trifluoressigsäure behandelt. Ausbeute 4.85 g.

4.8 g der oben gewonnenen Substanz werden analog Beispiel 30 L katalytisch hydriert und aufgearbeitet. Ausbeute 3.15 g. Zur weiteren Reinigung werden 800 mg der Substanz an einem hydroxypropylierten vernetzten Dextrangel in 70prozentigem Methanol chromatographiert. Ausbeute 465 mg, [α]_{D}²⁴ = -47.1° (c = 1, Wasser). Aminosäureanalyse: Glu (0.98), Val (1.00), Tyr (0.85), Lys (1.01) Arg (1.00), Gehalt an Peptidbase: ca. 80%.

### Beispiel 36

### Arg-Lys-Glu-Val-Tyr-NH-CH₂-CH(CH₃)₂, Acetat

### A) Z-Tyr-NH-CH₂-CH(CH₃)₂

Analog Beispiel 35 A werden 18.6 g (50 mmol) Z-Tyr(Bu^{t})-OH mit 5 ml (50 mmol) Isobutylamin kondensiert. Ausbeute 17.19 g (80%), Schmp. 114-115°, [α]_{D}²³ = -₃._{0°} (c = 1, Methanol).

### B) H-Tyr-NH-CH₂-CH(CH₃)₂ - HCl

17 g (ca. 40 mmol) Z-Tyr-NH-CH₂-CH(CH₃)₂ werden analog Beispiel 25 B katalytisch hydriert. Ausbeute 13.2 g (100%), Schmp. 172°, [α]_{D}²³ = +45.7° (c = 1, Methanol).

### C) Z-Val-Tyr-NH-CH₂-CH(CH₃)₂

Analog Beispiel 35 C werden 8.22 g (25 mmol) H-Tyr-NH-CH₂-CH(CH₃)₂-hydrochlorid und 6.27 g (25 mmol) Z-Val-OH kondensiert. Ausbeute 10.51 g (79%), Schmp. 207°, [α]_{D}²³ = -37.8° (c = 1, Dimethylformamid).

### D) H-Val-Tyr-NH-CH₂-CH(CH₃)₂ · HCl

9.95 g (19 mmol) Z-Val-Tyr(Bu^{t})-NH-CH₂-CH(CH₃)₂ werden analog Beispiel 35 D katalytisch hydriert. Ausbeute 8.13 g (100%), Schmp. 201-203°, [α]_{D}²³ = +24.6° (c = 1, Methanol).

### E) Z-Glu(OBu^{t})-Val-Tyr(Bu^{t})-NH-CH₂-CH(CH₃)₂

Analog Beispiel 35 E werden 7.7 g (18 mmol) H-Val-Tyr(Bu^{t})-NH-CH₂-CH(CH₃)₂ · HCI mit 9.83 g (19 mmol) Z-Glu(OBu^{t})-OTcp umgesetzt. Ausbeute 8.37 g (65%), Schmp. 173-174°, [α]D²² = -23.8° (c = 1, Dimethylformamid).

### F) H-Glu(OBu^{t})-Val-Tyr(Bu^{t})-NH-CH₂-CH(CH₃)₂ · HCI

7.82 g (11 mmol) Z-Glu(OBu^{t})-Val-Tyr(Bu^{t})-NH-CH₂-CH(CH₃)₂ werden analog Beispiel 35 D katalytisch hydriert. Die Substanz ist etwas ätherlöslich, sie wird deshalb nicht mit Äther verrieben. Ausbeute 6.16 g (91%), Schmp. 202-205° Zers., [α]_{D}²⁶ = -10.4° (c = 1, Methanol).

### G) Z-Lys(Boc)-Glu(OBu^{t})-Val-Tyr(Bu^{t})-NH-CH₂-CH(CH₃)₂

Analog Beispiel 30 G werden 5.82 g (9.5 mmol) H-Glu(OBu^{t})-Val-Tyr(Bu^{t})-NH-CH₂-CH(CH₃)₂ HCl mit 5.6 g (10 mmol) Z-Lys(Boc)-OTcp umgesetzt. Die Substanz wird zur Reinigung mit 100 ml Essigester aufgekocht. Dazu gibt man 100 ml Petroläther und saugt ab. Ausbeute 7.9 g (88%), Schmp. 209-211 °C, [α]_{D}²⁴ = -34.9° (c = 1, Methanol).

### H) H-Lys(Boc)-Glu(OBu^{t})-Val-Tyr(Bu^{t})-NH-CH₂-CH(CH₃)₂ · HCI

7.5 g (8 mmol) Z-Lys(Boc)-Glu(OBu^{t})-Val-Tyr(Bu^{t})-NH-CH₂-CH(CH₃)₂ werden analog Beispiel 25 B katalytisch hydriert. Die Substanz kristallisiert aus Äther. Ausbeute 6.0 g (89%), Schmp. 244-245°, [α]_{D}²⁴ = 25.9° (c = 1, Methanol).

### I) Z-Arg(Z₂)-Lys(Boc)-Glu(OBu^{t})-Val-Tyr-(Bu^{t})-NH-CH₂-CH(CH₃)₂

Analog Beispiel 30 I werden 5.46 g (6.5 mmol) H-Lys(Boc)-Glu(OBu^{t})-Val-Tyr(Bu^{t})-NH-CH₂-CH(CH₃)₂ · HCI mit 4.91 g (6.5 mmol) Z-Arg(Z₂)-OTcp umgesetzt. Die Substanz wird mit 150 ml Essigester ausgekocht und nach Zugabe von 150 ml Petroläther abgesaugt. Ausbeute 8.79 g (99%), Schmp. 214-216°, [α]_{D}²⁴ = -18.4° (c = 1, Eisessig).

### K) H-Arg-Lys-Glu-Val-Tyr-NH-CH₂-CH(CH₃)₂, Acetat

Analog Beispiel 30 K werden 8.18 g (6 mmol) mit 90prozentiger Trifluoressigsäure behandelt. Ausbeute 8.6 g (enthält noch Wasser und Trifluoressigsäure).

8 g der oben gewonnenen Substanz werden analog Beispiel 30 L katalytisch hydriert und aufgearbeitet. Ausbeute 3.98 g. Zur weiteren Reinigung werden 800 mg der Substanz an einem hydroxypropylierten vernetzten Dextrangel in 70prozentigem Methanol chromatographiert. Ausbeute 650 mg, [α]_{D}²² = -52.7° (c = 1, Methanol).

### Beispiel 37

### Arg-Lys-Glu-Val-Tyr-piperidid

### A) Z-Tyr(Bu^{t})-piperidid

Analog Beispiel 35 A werden 9.27 g (25 mmol) Z-Tyr(Bu^{t})-OH und 2.48 ml Piperidin (25 mmol) kondensiert. Ausbeute 10.1 g (92%) fast farbloses Öl.

### B) H-Tyr(Bu^{t})-piperidid · HCI

10.1 g (23 mmol) Z-Tyr(Bu^{t})-piperidid werden analog Beispiel 25 B katalytisch hydriert. Die Substanz kristallisiert aus Äther. Ausbeute 7.51 g (95%), Schmp. 205°, [α]_{D}²³ = +47.1° (c = 1, Methanol).

### C) Z-Val-Tyr(Bu^{t})-piperidid

Analog Beispiel 35 C werden 8.52 g (25 mmol) H-Tyr(Bu^{t})-piperidid · HCI und 6.27 g (25 mmol) Z-Val-OH kondensiert. Die Substanz kristallisiert aus Äther. Ausbeute 7.97 g (70%), Schmp. 142°C, [α]_{D}²³ = -32.8° (c = 1, Methanol).

### D) H-Val-Tyr(Bu^{t})-piperidid · HCl

7.47 g (16.5 mmol) Z-Val-Tyr(Bu^{t})-piperidid werden analog Beispiel 25 B katalytisch hydriert. Die Substanz wird scharf getrocknet. Sie kristallisiert nicht aus Äther. Ausbeute 5.92 g amorphe Substanz. Zersetzt sich bei etwa 174°C, [α]_{D}²³ = +25.2° (c = 1, Methanol).

### E) Z-Glu(OBu^{t})-Val-Tyr(Bu^{t})-piperidid

Analog Beispiel 30 E werden 5.5 g (12.5 mmol) H-Val-Tyr(Bu^{t})-piperidid . HCl mit 6.71 g (13 mmol) Z-Glu(OBu^{t})-OTcp umgesetzt. Die Substanz ist ölig und wird in Essigester geschüttelt. Zur Reinigung wird in Methylenchlorid an 250 g Kieselgel chromatographiert. Trichlorphenol wird mit Methylenchlorid eluiert. Das Peptid wird mit einer Mischung aus Methylenchlorid/Aceton wie 8 : 2 eluiert. Ausbeute 7.3 g (80%), [α]_{D}²⁴ = -41.4° (c = 1, Methanol), Schmp. 90-98° (amorph).

### F) H-Glu(OBu^{t})-Val-Tyr(Bu^{t})-piperidid · HCI

6.5 g (9 mmol) Z-Glu(OBu^{t})-Val-Tyr(Bu^{t})-piperidid werden analog Beispiel 25 B katalytisch hydriert. Die Substanz wird mit Äther verrieben. Ausbeute 5.16 g (92%) amorphe Substanz, [α]_{D}²³ = -18.9° (c = 1, Methanol).

### G) Z-Lys(Boc)-Glu(OBu^{t})-Val-Tyr(Bu^{t})-piperidid

Analog Beispiel 30 G werden 4.96 g (8 mmol) H-Glu(OBu^{t})-Val-Tyr(Bu^{t})-piperidid · HCI mit 4.76 g (8.5 mmol) Z-Lys(Boc)-OTcp umgesetzt. Kristallisiert aus Diisopropyläther/Petroläther. Ausbeute 6.5 g (85%), Schmp. 160-161 °C, [α]_{D}²³ = -40.1° (c = 1, Methanol).

### H) H-Lys(Boc)-Glu(OBut)-Val-Tyr(Bu^{t})-piperidid · HCI

6.0 g (6.3 mmol) Z-Lys(Boc)-Glu(OBu^{t})-Val-Tyr-(Bu^{t})-piperidid werden analog Beispiel 25 B katalytisch hydriert. Die Substanz wird mit Äther verrieben' und abgesaugt. Ausbeute 4.48 g (83%), Schmp. 171-174° unter Zerst., [α]_{D}²³ = -28.0° (c = 1, Methanol).

### I) Z-Arg(Z₂)-Lys(Boc)-Glu(OBu^{t})-Val-Tyr-(Bu^{t})-piperidid

Analog Beispiel 30 I werden 4.26 g (5 mmol) H-Lys(Boc)-Glu(OBu^{t})-Val-Tyr(Bu^{t})-piperidid · HCI.
mit 3.97 g (5.25 mmol) Z-Arg(Zz)-OTcp umgesetzt. Die Substanz wird mit Äther verrieben. Ausbeute 6.06 g (88%), Schmp. 177-178°, [α]_{D}²³ = -11.2° (c = 1, Dimethylformamid).

### K) H-Arg-Lys-Glu-Val-Tyr-piperidid, Acetat

Analog Beispiel 30 K werden 5.5 g (4 mmol) Z-Arg(Z₂)-Lys(Boc)-Glu(OBu^{t})-Val-Tyr(Bu^{t})-piperidid
mit 90prozentiger Trifluoressigsäure behandelt. Ausbeute 4.83 g.

4.55 g der oben gewonnenen Substanz werden analog Beispiel 30 L katalytisch hydriert und aufgearbeitet. Ausbeute 2.6 g. Zur weiteren Reinigung wurden 800 mg der Substanz an einem hydroxypropylierten vernetzten Dextrangel in 90prozentigem Methanol chromatographiert. Ausbeute 613 mg, [α]_{D}²⁷= -45.4° (c = 1, Wasser).

### Beispiel 38

### Arg-Lys-D-Aad-Val-Tyr-OMe, Acetat

### A) Z-D-Aad(OBu^{t})-Val-Tyr(Bu^{t})-OMe

Zu einer Lösung von 3.54 g (10 mmol) Z-D-Aad-(OBu^{t})-OH, 3.86 g (10 mmol) H-Val-Tyr(Bu^{t})-OMe · HCI und 1.35 g (10 mmol) 1-Hydroxybenzotriazol in 20 ml Dimethylformamid gibt man bei 0°C 1.28 ml (10 mmol) N-Äthylmorpholin und 2.1 g Dicyclohexylcarbodiimid. Man lässt 1 Stunde bei 0°C und anschliessend bei Raumtemperatur rühren. Man lässt über Nacht stehen und saugt anderntags den Niederschlag ab. Das Filtrat wird im Hochvakuum eingeengt und der Rückstand zwischen Essigester und Wasser verteilt. Die Essigesterphase wird nacheinander mit ges. NaHCO₃-Lösung, KHSO₄-Lösung und Wasser ausgeschüttelt, über Na₂SO₄ getrocknet und eingeengt. Der Rückstand kristallisiert aus Petroläther. Ausbeute 5.96 g. Zur weiteren Reinigung wurde die Substanz aus 90 ml Isopropyläther und 30 ml Isopropanol heiss umkristallisiert. Ausbeute 2.48 g, Schmp. 104-107°, [a]ₒ²³ = -11.5° (c = 1, Methanol).

### B) H-D-Aad(OBu^{t})-Val-Tyr(Bu^{t})-OMe . HCI

Zu einer Lösung von 2.3 g Z-D-Aad(OBu^{t})-Val-Tyr(Bu^{t})-OMe in 100 ml Methanol gibt man Pd/ Kohle-Katalysator und leitet unter Rühren und unter Zugabe von ca. 2N methanolischer Salzsäure bei pH 4.5 (Autotitrator) Wasserstoff durch die Lösung, bis keine methanolische Salzsäure mehr aufgenommen wird. Danach wird der Katalysator abgesaugt und das Filtrat eingeengt. Ausbeute 1.98 g, Schmp. 85-87°, [α]_{D}²² = -14.9° (c = 1, Methanol).

### C) Z-Lys(Boc)-D-Aad(OBu^{t})-Val-Tyr(Bu^{t})-OMe

Zu einer Lösung von 1.91 g H-D-Aad(OBu^{t})-Val-Tyr(Bu^{t})-Ome - HCI und 0.44 g 1-Hydroxybenzotriazol in 10 ml Dimethylformamid gibt man bei 0°C 0.41 ml N-Äthylmorpholin und 2 g Z-Lys(Boc)-OTcp. Man lässt eine Stunde bei 0°C und 3 Stunden bei Raumtemperatur stehen, engt die Lösung im Hochvakuum ein und arbeitet wie unter A auf. Kristallisiert aus Petroläther. Ausbeute 2.65 g, Schmp. 165°, [α]_{D}²² = -3.5° (c = 1, Methanol).

### D) H-Lys(Boc)-D-Aad(OBu^{t})-Val-Tyr(Bu^{t})-OMe - HCI

2.3 g Z-Lys(Boc)-D-Aad(OBu^{t})-Val-Tyr(Bu^{t})-OMe werden analog B katalytisch hydriert. Der Rückstand wird mit Petroläther verrieben. Ausbeute 1.38 g, Schmp. 115-117°, [a]ₒ²² = +3.2° (c = 1, Methanol).

### E) Z-Arg(Z₂)-Lys(Boc)-D-Aad(OBu^{t})-Val-Tyr(Bu^{t})-OMe

Zu einer Lösung von 1.2 g H-Lys(Boc)-D-Aad(O-Bu^{t})-Val-Tyr(Bu^{t})-OMe · HCI und 0.2 1-Hydroxybenzotriazol in 10 ml Dimethylformamid gibt man bei 0°C 0.19 ml N-Äthylmorpholin und 1.12g Z-Arg(Z₂)-OTcp. Man lässt 1 Stunde bei 0°C und 4 Stunden bei Raumtemperatur rühren. Mit 100 ml Eiswasser unter Zusatz von etwa 5 ml ges. NaH-CO₃-Lösung wird ein Niederschlag ausgefällt, der mit KHSO₄-Lösung und Wasser gut gewaschen wird. Die Substanz wird über P₂O₅ getrocknet und mit 30 ml Äther verrührt. Die Substanz wird abgesaugt und getrocknet. Ausbeute 1.74 g, Schmp. 167-170°, [α]_{D}²² = -3.1° (c = 1, 90prozentige Essigsäure).

### F) H-Arg-Lys(Boc)-D-Aad(OBu^{t})-Val-Tyr-(But)-OMe · HCI

1.5 g Z-Arg(Z₂)-Lys(Boc)-D-Aad(OBu^{t})-Val-Tyr-(Bu^{t})-OMe werden analog B katalytisch hydriert. Der Rückstand wird mit Äther verrieben und abgesaugt. Ausbeute 1.05 g, Schmp. 112-114°, [α]_{D}²² = +8.7° (c = 1, 90prozentige Essigsäure).

### G) H-Arg-Lys-D-Aad-Val-Tyr-OMe, Acetat

1 g H-Arg-Lys(Boc)-D-Aad(OBu^{t})-Val-Tyr(Bu^{t})-OMe . 2 HCI wird in 10 ml 90prozentiger Trifluoressigsäure gelöst. Man lässt eine Stunde bei Raumtemperatur stehen, engt im Vakuum ein und destilliert mit Methanol nach. Der Rückstand wird in Wasser gelöst und über einen basischen lonenaustauscher in Acetatform chromatographiert. Das Eluat wird gefriergetrocknet und anschliessend an einem hydroxypropylierten vernetzten Dextrangel in 90prozentigem Methanol chromatographiert. Ausbeute 340 mg, Aminosäureanalyse (Hydrolyse: 24 Stunden bei 120°C): Aad (0.81), Val (0.94), Tyr (0.84), Lys (1.00), Arg (1.00), Gehalt an Peptidbase: ca. 80%.

### Beispiel 39

### Arg-Lys-D-Asp-Val-Tyr-OMe, Acetat

### A) Z-D-Asp(OBu^{t})-Val-Tyr(Bu^{t})-OMe

Analog Beispiel 38 A werden 6.46 g (20 mmol) Z-D-Asp(OBu^{t})-OH mit 7.73 g (20 mmol) H-Val-Tyr-(But)-OMe · HCI in 50 ml Dimethylformamid kondensiert. Ausbeute 11.2 g (85%), Schmp. 90-92°, [α]_{D}²⁷ = -3.5° (c = 1, Methanol).

### B) H-D-Asp(OBu^{t})-Val-Tyr(Bu^{t})-OMe · HCl

Analog Beispiel 38 B werden 10.49 g (16 mmol) Z-D-Asp(OBu^{t})-Val-Tyr(Bu^{t})-OMe katalytisch hydriert. Der Rückstand wird in Äther gelöst. Von einem unlöslichen Rückstand wird filtriert. Das Filtrat wird eingeengt und im Hochvakuum getrocknet. Ausbeute 8.79 g (98%, amorph, [α]_{D}²⁷ = -10.4° (c = 1, Methanol).

### C) Z-Lys(Boc)-D-Asp(OBu^{t})-Val-Tyr(Bu^{t})-OMe

Analog Beispiel 38 C werden 8.37 g (15 mmol) H-D-Asp(OBu^{t})-Val-Tyr(Bu^{t})-OMe · HCI mit 8.68 g (15.5 mmol) Z-Lys(Boc)-OTcp umgesetzt. Die Substanz kristallisiert aus Petroläther. Ausbeute 11.52 g (87%), Schmp. 142-145°, [α]_{D}²⁷ = +3.4° (c = 1, Methanol).

### D) H-Lys(Boc)-D-Asp(OBu^{t})-Val-Tyr-(Bu^{t})-OMe · HCI

Analog Beispiel 38 B werden 11.05 g (12.5 mmol) Z-Lys(Boc)-D-Asp(OBu^{t})-Val-Tyr(Bu^{t})-OMe katalytisch hydriert. Ausbeute 9.1 g (92%), amorph, [α]_{D}²⁷ = +17.6° (c = 1, Methanol).

### E) Z-Arg(Z₂)-Lys(Boc)-D-Asp(OBu^{t})-Val-Tyr(Bu^{t})-OMe

Analog Beispiel 38 E werden 1.57 g (2 mmol) H-Lys(Boc)-D-Asp(OBu^{t})-Val-Tyr(Bu^{t})-OMe · HCl mit 1.6 g (2.2 mmol) Z-Arg(Z₂)-OTcp umgesetzt und analog Beispiel 38 A aufgearbeitet. Zur Reinigung wird die Substanz über 80 g Kieselgel chromatographiert. Elutionsmittel ist Methylenchlorid/Aceton wie 7: 3. Der Rückstand wird mit Petroläther verrieben. Ausbeute 1.97 g (75%), Schmp. 150-154°, [α]_{D}²⁵ = +7.2° (c = 1, Dimethylformamid).

### F) H-Arg-Lys-D-Asp-Val-Tyr-OMe, Acetat

1.3 g (1 mmol) Z-Arg(Z_{z})-Lys(Boc)-D-Asp(OBu^{t})-Val-Tyr(Bu^{t})-OMe werden in 5 ml 90prozentiger Trifluoressigsäure gelöst. Man lässt eine Stunde bei Raumtemperatur stehen und engt im Vakuum ein. Der Rückstand wird mit Wasser verrieben, abgesaugt und getrocknet. Die Substanz, die noch etwas Wasser und Trifluoressigsäure enthält, wird in 10-20 ml 90prozentiger Essigsäure gelöst. Dazu gibt man Pd-Kohle-Katalysator und leitet Wasserstoff durch die Lösung, bis kein CO_{z} mehr entweicht. Der Katalysator wird abgesaugt, das Filtrat eingeengt und der Rückstand in Wasser gelöst. Die Lösung wird an einem schwach basischen Ionenaustauscher in Acetatform (30 x 2 cm) chromatographiert. Elution mit Wasser. Das peptidhaltige Eluat wird gefriergetrocknet und zur weiteren Reinigung an einem hydroxypropylierten vernetzten Dextrangel in 90prozentigem Methanol chromatographiert. Ausbeute 430 mg. Aminosäureanalyse: Asp (1.00), Val (1.1), Tyr (0.9), Lys (1.09), Arg (0.98), Gehalt an Peptidbase. ca. 78%.

### Beispiel 40

### Arg-Lys-D-Glu-Val-Tyr-OMe, Acetat

### A) Z-D-Glu(OBu^{t})-Val-Tyr(Bu^{t})-OMe

Analog Beispiel 38 A werden 4.04 g (12 mmol) Z-D-Glu(OBu^{t})-OH mit 4.64 g (12 mmol) H-Val-Tyr-(Bu^{t})-OMe · HCI in 25 ml Dimethylformamid kondensiert. Ausbeute 6.72 g. Zur weiteren Reinigung wird die Substanz an Kieselgel chromatographiert. Elutionsmittel ist eine Mischung aus Methylenchlorid und Aceton wie 9:1. Ausbeute 5.63 g (70%), Schmp. 100-104°, [α]_{D}²⁷ = -8.4°n (c = 1, Methanol).

### B) H-D-Glu(OBu^{t})-Val-Tyr(Bu^{t})-OMe · HCl

Analog Beispiel 38 B werden 5.4 g (8 mmol) Z-D-Glu(OBu^{t})-Val-Tyr(Bu^{t})-OMe katalytisch hydriert. Der Rückstand wird im Hochvakuum getrocknet. Ausbeute 4.57 g (100%). Die Substanz ist amorph.

### C) Z-Lys(Boc)-D-Glu(OBu^{t})-Val-Tyr(Bu^{t})-OMe

Analog Beispiel 38 C werden 4.57 g (8 mmol) H-D-Glu(OBu^{t})-Val-Tyr(Bu^{t})-OMe · HCl mit 4.76 g (8.5 mmol) Z-Lys(Boc)-OTcp umgesetzt. Mit 250 ml Wasser, dem 10 ml gesättigte NaHCO₃-Lösung zugesetzt werden, wird die Substanz aus der Dimethylformamidlösung ausgefällt. Der Niederschlag wird abgesaugt und gut mit Wasser gewaschen und getrocknet. Anschliessend wird die Substanz mit Diäthyläther verrieben, abgesaugt und getrocknet. Ausbeute 6.22 g (87%), Schmp. 164-167°, [α]_{D}²⁵ = -1° (c = 1, Dimethylformamid).

### D) H-Lys(Boc)-D-Glu(OBu^{t})-Val-Tyr-(Bu^{t})-OMe · HCI

Analog Beispiel 38 B werden 6.01 g (6.6 mmol) Z-Lys(Boc)-D-Glu(OBu^{t})-Val-Tyr(Bu^{t})-OMe katalytisch hydriert. Ausbeute 5.28 g (100%), [α]_{D}²⁴ = +8.6° (c = 1, Methanol).

### E) Z-Arg(Z₂)-Lys(Boc)-D-Glu(OBu^{t})-Val-Tyr(Bu^{t})-OMe

Analog Beispiel 38 E werden 1.6 g (2 mmol) H-Lys(Boc)-D-Glu(OBu^{t})-Vat-Tyr(Bu^{t})-OMe · HCl mit 1.66 g (2.2 mmol) Z-Arg(Z_{z})-OTcp umgesetzt. Aufgearbeitet wird analog Beispiel 40 C. Ausbeute 2.44 g (94%), Schmp. 153-156°, [α]_{D}²⁵ = 0 (c = 1, Dimethylformamid).

### F) H-Arg-Lys-D-Glu-Val-Tyr-OMe, Acetat

1.32 g (1 mmol) Z-Arg(Zz)-Lys(Boc)-D-Glu(O-Bu^{t})-Val-Tyr(Bu^{t})-OMe werden analog Beispiel 39 F umgesetzt. Ausbeute 445 mg.

Aminosäureanalyse: Glu (1.00), Val (0.96), Tyr (0.91), Lys (1.04), Arg (0:95), Gehalt an Peptidbase: ca. 80%.

## Patentansprüche (Patentansprüche für folgende(n) Vertragsstaat(en) : BE CH DE FR GB IT LI NL SE)

1. Peptid der Formel 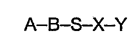 in welcher bedeutet
A Arginin, Lysin, Ornithin oder Homoarginin, jeweils in L- oder D-Konfiguration, ω-Amino-, -Guanidino- oder -Dimethylamino-alkanoyl mit 3-6 C-Atomen und gegebenenfalls einer a-Aminogruppe in D- oder L-Konfiguration, die ihrerseits Alkanoyl mit 1-6 C-Atomen, Aroyl mit 7-11 C-Atomen, Cycloalkanoyl mit bis zu 2 Alkyl- und 5-7 Cycloalkyl-C-Atomen, Aralkanoyl mit bis zu insgesamt 9 C-Atomen, wobei eine -CH_{z}-Gruppe durch -0- oder -S- ersetzt sein kann, Alkyl- oder Aralkyl-oxycarbonyl mit bis zu 7 C-Atomen oder Succinoyl, Succinamoyl, Glutaroyl, Glutaminyl, Pyroglutamyl, Phthaloyl, Phthalamidyl oder2-Carboxy- benzöyl tragen kann.
B eine basische Aminosäure, vorzugsweise L-Lysin, L-Arginin, L-Homoarginin oder L-Ornithin
X L-Valin oder L-Isoleucin und
Y eine L- bzw. D-Aminosäure mit hydrophober Seitenkette, deren Ester, Amid, Alkylamid mit 1-6 C-Atomen oder Aralkylamid mit 7-10 C-Atomen, Alkylamid oder Alkylester mit 1-6 C-Atomen oder Aralkylamid oder Aralkylester mit 7-10 C-Atomen
S Glu, D-Glu, D-Asp oder D-a-Aminoadipin-S Glu, und dessen Salze.

2.Peptid der Formel 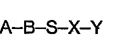 in welcher bedeutet
A Arginin, Lysin, Ornithin oder Homoarginin, jeweils in L- oder D-Konfiguration, ω-Amino-, -Guanidino- oder -Dimethylamino-alkanoyl mit 3-6 C-Atomen und gegebenenfalls einer a-Aminogruppe in D- oder L-Konfiguration, die ihrerseits Alkanoyl mit 1-6 C-Atomen, Aroyl mit 7-11 C-Atomen, Cycloalkanoyl mit bis zu 2 Alkyl- und 5-7 Cycloalkyl-C-Atomen, Aralkanoyl mit bis zu insgesamt 9 C-Atomen, wobei eine -CH₂-Gruppe durch -O- oder -S- ersetzt sein kann, Alkyl- oder Aralkyl-oxycarbonyl mit bis zu 7 C-Atomen oder Succinoyl, Succinamoyl, Glutaroyl, Glutaminyl, Pyroglutamyl, Phthaloyl, Phthalamidyl oder 2-Carboxy- benzoyl tragen kann
B eine basische Aminosäure, vorzugsweise L-Lysin, L-Arginin, L-Homoarginin oder L-Ornithin
X L-Valin oder L-lsoleucin und
Y eine L- bzw. D-Aminosäure mit hydrophober Seitenkette, deren Ester, Amid, Alkylamid mit 1-6 C-Atomen oder Aralkylamid mit 7-10 C-Atomen, Alkylamid oder Alkylester mit 1-6 C-Atomen oder Aralkylamid oder Aralkylester mit 7-10 C-Atomen S_{'}Glu, und dessensalze.

3. Peptid der Formel 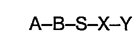 in welcher bedeutet
A Arginin, Lysin, Ornithin oder Homoarginin, jeweils in L- oder D-Konfiguration, m-Amino-, -Guanidino- oder -Dimethylamino-alkanoyl mit 3-6 C-Atomen und gegebenenfalls einer a-Aminogruppe in D- oder L-Konfiguration, die ihrerseits Alkanoyl mit 1-6 C-Atomen, Aroyl mit 7-11 C-Atomen, Cycloalkanoyl mit bis zu 2 Alkyl- und 5-7 Cycloalkyl-C-Atomen, Aralkanoyl mit bis zu insgesamt 9 C-Atomen, wobei eine-CH₂-Gruppe durch -0- oder -S- ersetzt sein kann, Alkyl- oder Aralkyl-oxycarbonyl mit bis zu 7 C-Atomen oder Succinoyl, Succinamoyl, Glutaroyl, Glutaminyl, Pyroglutamyl, Phthaloyl, Phthalamidyl oder2-Carboxy- benzoyl tragen kann
B eine basische Aminosäure, vorzugsweise L-Lysin, L-Arginin, L-Homoarginin oder L-Ornithin
X L-Valin oder L-Isoleucin und
Y eine L- bzw. D-Aminosäure mit hydrophober Seitenkette, deren Ester, Amid, Alkylamid mit 1-6 C-Atomen oder Aralkylamid mit 7-10 C-Atomen, Alkylamid oder Alkylester mit 1-6 C-Atomen oder Aralkylamid oder Aralkylester mit 7-10 C-Atomen
S D-Glu, D-Asp oder D-a-Aminoadipinsäure, wobei Peptide der Formeln A-Lys-S-X-Y und A-D-Lys-S-X-Y, in denen A gegebenenfalls mit (C₁-C,)-Alkanoyl oder Gln substituiertes Arginin, Homoarginin oder (C₃-C₆)-ω-Guanidinoalkanoyl bedeutet und Y gleichzeitig Tyr oder D-Tyr bedeutet, sowie deren Amid, (C₁-C₆)-Alkylamid oder (C₁-C₆)-Alkylester ausgenommen sind, und dessen Salze.

4. Peptid gemäss einem der Ansprüche 1 und 2 mit Formel Arg-Lys-Glu-Val-Trp-OMe.

5. Peptid gemäss einem der Ansprüche 1 und 2 mit Formel Arg-Lys-Glu-Val-Tyr-OMe.

6. Peptid gemäss einem der Ansprüche 1 und 2 mit der Formel D-Lys-Arg-Glu-Val-Tyr-OMe.

7. Peptid gemäss Anspruch 1 mit der Formel Arg-Lys-D-Aad-Val-Tyr-OMe.

8. Peptid gemäss Anspruch 1 mit der Formel Arg-Lys-D-Glu-Val-Tyr-OMe.

9. Verfahren zur Herstellung der Peptide nach einem der Ansprüche 1-8, dadurch gekennzeichnet, dass man Aminosäuresequenzen der Formel 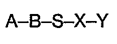 in der A, B, S, X und Y die in den Ansprüchen 1-8 wiedergegebenen Bedeutungen haben, nach Methoden der Peptidsynthese aufbaut.

10. Peptid gemäss einem der Ansprüche 1-8 zur Verwendung als Mittel zur Beeinflussung der Reifung der T-Lymphozyten.

11. Peptid gemäss einem der Ansprüche 1-8 zur Verwendung als Heilmittel.

## Patentansprüche (Patentansprüche für folgende(n) Vertragsstaat(en) : AT ')

1. Verfahren zur Herstellung neuer Peptide der allgemeinen Formel 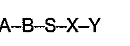 in welcher bedeutet
A Arginin, Lysin, Ornithin oder Homoarginin, jeweils in L- oder D-Konfiguration, m-Amino-, -Guanidino- oder -Dimethylamino-alkanoyl mit 3-6 C-Atomen und gegebenenfalls einer a-Aminogruppe in D- oder L-Konfiguration, die ihrerseits Alkanoyl mit 1-6 C-Atomen, Aroyl mit 7-11 C-Atomen, Cycloalkanoyl mit bis zu 2 Alkyl- und 5-7 Cycloalkyl-C-Atomen, Aralkanoyl mit bis zu insgesamt 9 C-Atomen, wobei eine -CH₂-Gruppe durch -0- oder -S- ersetzt sein kann, Alkyl- oder Aralkyl-oxycarbonyl mit bis zu 7 C-Atomen oder Succinoyl, Succinamoyl, Glutaroyl, Glutaminyl, Pyroglutamyl, Phthaloyl, Phthalamidyl oder 2-Carboxybenzoyl tragen kann
B eine basische Aminosäure, vorzugsweise L-Lysin, L-Arginin, L-Homoarginin oder L-Ornithin
X L-Valin oder L-Isoleucin und
Y eine L- bzw. D-Aminosäure mit hydrophober Seitenkette, deren Ester, Amid, Alkylamid mit 1-6 C-Atomen oder Aralkylamid mit 7-10 C-Atomen, Alkylamid oder Alkylester mit 1-6 C-Atomen oder Aralkylamid oder Aralkylester mit 7-10 C-Atomen,
S Glu, D-Glu, D-Asp oder D-a-Aminoadipinsäure, und deren Salzen, dadurch gekennzeichnet, dass man Aminosäuresequenzen der genannten Formel nach Methoden der Peptidsynthese aufbaut.

2. Verfahren zur Herstellung neuer Peptide der allgemeinen Formel 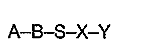 in welcher bedeutet
A Arginin, Lysin, Ornithin oder Homoarginin, jeweils in L- oder D-Konfiguration, ω-Amino-, -Guanidino- oder -Dimethylamino-alkanoyl mit 3-6 C-Atomen und gegebenenfalls einer a-Aminogruppe in D- oder L-Konfiguration, die ihrerseits Alkanoyl mit 1-6 C-Atomen, Aroyl mit 7-11 C-Atomen, Cycloalkanoyl mit bis zu 2 Alkyl- und 5-7 Cycloalkyl-C-Atomen, Aralkanoyl mit bis zu insgesamt 9 C-Atomen, wobei eine-CH₂-Gruppe durch -0- oder -S- ersetzt sein kann, Alkyl- oder Aralkyl-oxycarbonyl mit bis zu 7 C-Atomen oder Succinoyl, Succinamoyl, Glutaroyl, Glutaminyl, Pyroglutamyl, Phthaloyl, Phthalamidyl oder 2-Carboxybenzoyl tragen kann
B eine basische Aminosäure, vorzugsweise L-Lysin, L-Arginin, L-Homoarginin oder L-Ornithin
X L-Valin oder L-Isoleucin und
Y eine L- bzw. D-Aminosäure mit hydrophober Seitenkette, deren Ester, Amid, Alkylamid mit 1-6 C-Atomen oder Aralkylamid mit 7-10 C-Atomen, Alkylamid oder Alkylester mit 1-6 C-Atomen oder Aralkylamid oder Aralkylester mit 7-10 C-Atomen,
S Glu, und deren Salzen, dadurch gekennzeichnet, dass man Aminosäuresequenzen der genannten Formel nach Methoden der Peptidsynthese aufbaut und diese Peptide gegebenenfalls in ihre Salze überführt.

3. Verfahren zur Herstellung neuer Peptide der allgemeinen Formel 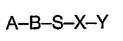 in welcher bedeutet
A Arginin, Lysin, Ornithin oder Homoarginin, jeweils in L- oder D-Konfiguration, co-Amino-, -Guanidino- oder -Dimethylamino-alkanoyl mit 3-6 C-Atomen und gegebenenfalls einer a-Aminogruppe in D- oder L-Konfiguration, die ihrerseits Alkanoyl mit 1-6 C-Atomen, Aroyl mit 7-11 C-Atomen, Cycloalkanoyl mit bis zu 2 Alkyl- und 5-7 Cycloalkyl-C-Atomen, Aralkanoyl mit bis zu insgesamt 9 C-Atomen, wobei eine -CH₂-Gruppe durch -0- oder -S- ersetzt sein kann, Alkyl- oder Aralkyl-oxycarbonyl mit bis zu 7 C-Atomen oder Succinoyl, Succinamoyl, Glutaroyl, Glutaminyl, Pyroglutamyl, Phthaloyl, Phthalamidyl oder 2-Carboxybenzoyl tragen kann
B eine basische Aminosäure, vorzugsweise L-Lysin, L-Arginin, L-Homoarginin oder L-Ornithin
X L-Valin oder L-Isoleucin und
Y eine L- bzw. D-Aminosäure mit hydrophober Seitenkette, deren Ester, Amid, Alkylamid mit 1-6 C-Atomen oder Aralkylamid mit 7-10 C-Atomen, Alkylamid oder Alkylester mit 1-6 C-Atomen oder Aralkylamid oder Aralkylester mit 7-10 C-Atomen,
S D-Glu, D-Asp oder D-a-Aminoadipinsäure, wobei das Verfahren zur Herstellung von Peptiden der Formeln A-Lys-S-X-Y und A-D-Lys-S-X-Y, in denen A gegebenenfalls mit (C₁-C₆)-Alkanoyl oder Gin substituiertes Arginin, Homoarginin oder (C₃-C₆)-ω-Guanidino-alkanoyl bedeutet und Y gleichzeitig Tyr oder D-Tyr bedeutet, sowie deren Amid, (C₁-C₆)-Alkylamid oder (C₁-C₆)-Alkylester ausgenommen sind, und deren Salzen, dadurch gekennzeichnet, dass man Aminosäuresequenzen der genannten Formel nach Methoden der Peptidsynthese aufbaut und diese Peptide gegebenenfalls in ihre Salze überführt.

4. Verfahren gemäss einem der Ansprüche 1 und 2, dadurch gekennzeichnet, dass Arg-Lys-Glu-Val-Trp-OMe hergestellt wird.

5. Verfahren gemäss einem der Ansprüche 1 und 2, dadurch gekennzeichnet, dass Arg-Lys-Glu-Val-Tyr-OMe hergestellt wird.

6. Verfahren gemäss einem der Ansprüche 1 und 2, dadurch gekennzeichnet, dass D-Lys-Arg-Glu-Val-Tyr-OMe hergestellt wird.

7. Verfahren gemäss Anspruch 1, dadurch gekennzeichnet, dass Arg-Lys-D-Aad-Val-Tyr-OMe hergestellt wird.

8. Verfahren gemäss Anspruch 1, dadurch gekennzeichnet, dass Arg-Lys-D-Glu-Val-Tyr-OMe hergestellt wird.

9. Verfahren gemäss einem der Ansprüche 1-8 zur Herstellung eines Peptids zur Verwendung als Mittel zur Beeinflussung der Reifung der T-Lymphozyten.

10. Verfahren gemäss einem der Ansprüche 1-8 zur Herstellung eines Peptids zur Verwendung als Heilmittel.

## Claims (Claims for the following Contracting State(s) : s: BE, CH, LI, DE, FR, GB, IT, NL, SE)

1. A peptide of the formula 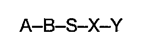 in which A denotes arginine, lysine, ornithine or homoarginine, in each case in the L- or D-configuration, or co-aminoalkanoyl, ω-guanidino- alkanoyl or ω-dimethylaminoalkanoyl which has 3-6 C atoms and optionally contains an a-amino group in the D- or L-configuration which in turn can carry alkanoyl having 1-6 C atoms, aroyl having 7-11 C atoms, cycloalkanoyl having up to 2 alkyl C atoms and 5-7 cycloalkyl C atoms, aralkanyol having up to 9 C atoms altogether, wherein a -CH_{z}- group can be replaced by -0- or -S-, alkyloxycarbonyl or aralkyloxycarbonyl having up to 7 C atoms or succinoyl, succinamoyl, glutaroyl, glutaminyl, pyroglutamyl, phthaloyl, phthalamidyl or 2-carboxybenzoyl, B denotes a basic aminoacid, preferably L-lysine, L-arginine, L-homo-arginine or L-ornithine, X denotes L-valine or L-isoleucine and Y denotes an L- or D-aminoacid having a hydrophobic side chain or an ester, amide, alkylamide or alkyl ester having 1-6 C atoms or aralkylamide or aralkyl ester having 7-10 C atoms, thereof and S denotes Glu, D-Glu, D-Asp or D-a-aminoadipic acid, and the salts thereof.

2. A peptide of the formula 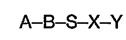 in which A denotes arginine, lysine, ornithine or homoarginine, in each case in the L- or D-configuration, or co-aminoalkanoyl, ω-guanidino- alkanoyl or co-dimethylaminoalkanoyl which has 3-6 C atoms and optionally contains an a-amino group in the D- or L-configuration which in turn can carry alkanoyl having 1-6 C atoms, aroyl having 7-11 C atoms, cycloalkanoyl having up to 2 alkyl C atoms and 5-7 cycloalkyl C atoms, aralkanoyl having up to 9 C atoms altogether, wherein a -CH_{z-} group can be replaced by-0- or-S-, alkyloxycarbonyl or aralkyloxycarbonyl having up to 7 C atoms or succinoyl, succinamoyl, glutaroyl, glutaminyl, pyroglutamyl, phthaloyl, phthalamidyl or 2-carboxybenzoyl, B denotes a basic aminoacid, preferably L-lysine, L-arginine, L-homo-arginine or L-ornithine, X denotes L-valine or L-isoleucine and Y denotes an L- or D-aminoacid having a hydrophobic side chain or an ester, amide, alkylamide or alkyl ester having 1-6 C atoms or aralkylamide or aralkyl ester having 7-10 C atoms, thereof and S denotes Glu, and the salts thereof.

3. A peptide of the formula 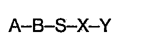 in which A denotes arginine, lysine, ornithine or homoarginine, in each case in the L- or D-configuration, or ω-aminoalkanoyl, m-guanidino- alkanoyl or ω-dimethylaminoalkanoyl which has 3-6 C atoms and optionally contains an a-amino group in the D- or L-configuration which in turn can carry alkanoyl having 1-6 C atoms, aroyl having 7-11 C atoms, cycloalkanoyl having up to 2 alkyl C atoms and 5-7 cycloalkyl C atoms, aralkanoyl having up to 9 C atoms altogether, wherein a-CH₂ group can be replaced by-O-or-S-, alkyloxycarbonyl or aralkyloxycarbonyl having up to 7 C atoms or succinoyl, succinamoyl, glutaroyl, glutaminyl, pyroglutamyl, phthaloyl, phthalamidyl or 2-carboxybenzoyl, B denotes a basic aminoacid, preferably L-lysine, L-arginine, L-homo-arginine or L-ornithine, X denotes L-valine or L-isoleucine and Y denotes an L- or D-aminoacid having a hydrophobic side chain or an ester, amide, alkylamide or alkyl ester having 1-6 C atoms or aralkylamide or aralkyl ester having 7-10 C atoms, thereof and S denotes D-Glu, D-Asp or D-a-aminoadipic acid, with the proviso that peptides of the formulae A-Lys-S-X-Y and A-D-Lys-S-X-Y, in which A denotes arginine, homoarginine or (C₃-C₆)-ω-guani- dinoalkanoyl, each optionally substituted by (C₁-Cₛ)-alkanoyl or Gln and Y denotes Tyr or D-Tyr, or the amide, (C₁-C₆)-alkylamide or (C₁-C₆)-alkylester thereof, are excluded; and the salts thereof.

4. Peptide according to one of Claims 1 and 2 having the formula Arg-Lys-Glu-Val-Trp-OMe.

5. Peptide according to one of Claims 1 and 2 having the formula Arg-Lys-Glu-Val-Tyr-OMe.

6. Peptide according to one of Claims 1 and 2 having the formula D-Lys-Arg-Glu-Val-Tyr-OMe.

7. Peptide according to Claim 1 having the formula Arg-Lys-D-Aad-Val-Tyr-OMe.

8. Peptide according to Claim 1 having the formula Arg-Lys-D-Glu-Val-Tyr-OMe.

9. A process for the preparation of peptides according to anyone of Claims 1-8, which comprises synthesizing, in accordance with methods of peptide synthesis, aminoacid sequences of the formula 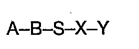 in which A, B, S, X and Y have the meaning given in Claims 1-8.

10. Peptide according to anyone of Claims 1-8 for use as agent for influencing the maturing of T-lymphocytes.

11. Peptide according to anyone of Claims 1-8 for use as a medicament.

## Claims (Claims for the following Contracting State(s) : Austria)

1. A process for the preparation of new peptides of the general formula 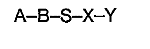 in which A denotes arginine, lysine, ornithine or homoarginine, in each case in the L- or D-configuration, or ω-aminoalkanoyl, ω-guanidino- alkanoyl or ω-dimethylaminoalkanoyl which has 3-6 C atoms and optionally contains an a-amino group in the D- or L-configuration which in turn can carry alkanoyl having 1-6 C atoms, aroyl having 7-11 C atoms, cycloalkanoyl having up to 2 alkyl C atoms and 5-7 cycloalkyl C atoms, aralkanoyl having up to 9 C atoms altogether, wherein a -CH_{z} group can be replaced by -O- or -S-, alkyloxycarbonyl or aralkyloxycarbonyl having up to 7 C atoms or succinoyl, succinamoyl, glutaroyl, glutaminyl, pyroglutamyl, phthaloyl, phthalamidyl or 2-carboxybenzoyl, B denotes a basic aminoacid, preferably L-lysine, L-arginine, L-homo-arginine or L-ornithine, X denotes L-valine or L-isoleucine and Y denotes an L- or D-aminoacid having a hydrophobic side chain or an ester, amide, alkylamide or alkyl ester having 1-6 C atoms or aralkylamide or aralkyl ester having 7-10 C atoms, thereof and S denotes Glu, D-Glu, D-Asp or D-a-aminoadipic acid and the salts thereof, which comprises synthesizing, in accordance with methods of peptide synthesis, aminoacid sequences of the said formula.

2. A process for the preparation of new peptides of the general formula 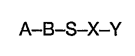 in which A denotes arginine, lysine, ornithine or homoarginine, in each case in the L- or D-configuration, or m-aminoalkanoyl, m-guanidino- alkanoyl or ω-dimethylaminoalkanoyl which has 3-6 C atoms and optionally contains an a-amino group in the D- or L-configuration which in turn can carry alkanoyl having 1-6 C atoms, aroyl having 7-11 C atoms, cycloalkanoyl having up to 2 alkyl C atoms and 5-7 cycloalkyl C atoms, aralkanoyl having up to 9 C atoms altogether, wherein a -CHz- group can be replaced by -O- or -S-, alkyloxycarbonyl or aralkyloxycarbonyl having up to 7 C atoms or succinoyl, succinamoyl, glutaroyl, glutaminyl, pyroglutamyl, phthaloyl, phthalamidyl or 2-carboxybenzoyl, B denotes a basic aminoacid, preferably L-lysine, L-arginine, L-homo-arginine or L-ornithine, X denotes L-valine or L-isoleucine and Y denotes an L- or D-aminoacid having a hydrophobic side chain or an ester, amide, alkylamide or alkyl ester having 1-6 C atoms or aralkylamide or aralkyl ester having 7-10 C atoms, thereof and S denotes Glu, and the salts thereof, which comprises synthesizing, in accordance with methods of peptide synthesis, aminoacid sequences of the said formula.

3. A process for the preparation of new peptides of the general formula 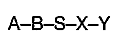 in which A denotes arginine, lysine, ornithine or homo-arginine, in each case in the L- or D-configuration, or ω-aminoalkanoyl, ω-guanidino- alkanoyl or ω-dimethylaminoalkanoyl which has 3-6 C atoms and optionally contains an a-amino group in the D- or L-configuration which in turn can carry alkanoyl having 1-6 C atoms, aroyl having 7-11 C atoms, cycloalkanoyl having up to 2 alkyl C atoms and 5-7 cycloalkyl C atoms, aralkanoyl having up to 9 C atoms altogether, wherein a -CH₂- group can be replaced by -O- or -S-, alkyloxycarbonyl or aralkyloxycarbonyl having up to 7 C atoms or succinoyl, succinamoyl, glutaroyl, glutaminyl, pyroglutamyl, phthaloyl, phthalamidyl or 2-carboxybenzoyl, B denotes a basic aminoacid, preferably L-lysine, L-arginine, L-homo-arginine or L-ornithine, X denotes L-valine or L-isoleucine and Y denotes an L- or D-aminoacid having a hydrophobic side chain or an ester, amide, alkylamide or alkyl ester having 1-6 C atoms or aralkylamide or aralkyl ester having 7-10 C atoms, thereof and S denotes D-Glu, D-Asp or D-a-aminoadipic acid, with the proviso that peptides of the formulae A-Lys-S-X-Y and A-D-Lys-S-X-Y, in which A denotes arginine, homoarginine or (C₃-C₆)-ω-guani- dinoalkanoyl, each optionally substituted by (C₁-C₆)-alkanoyl or Gin and Y denotes Tyr or D-Tyr, or the amide, (C₁-C₆)-alkylamide or (C₁-C₆)-alkylester thereof, are excluded; and the salts thereof;
which comprises synthesizing, in accordance with methods of peptide synthesis, aminoacid sequences of the said formula.

4. A process according to one of Claims 1 and 2, characterized in that Arg-Lys-Glu-Val-Trp-OMe is prepared.

5. A process according to one of Claims 1 and 2, characterized in that Arg-Lys-Glu-Val-Tyr-OMe is prepared.

6. A process according to one of Claims 1 and 2, characterized in that D-Lys-Arg-Glu-Val-Tyr-OMe is prepared.

7. A process according to Claim 1, characterized in that Arg-Lys-D-Aad-Val-Tyr-OMe is prepared.

8. A process according to Claim 1, characterized in that Arg-Lys-D-Glu-Val-Tyr-OMe is prepared.

9. A process according to anyone of Claims 1-8 for preparing a peptide for use as agent for influencing the maturing of T-lymphocytes.

10. A process according to anyone of Claims 1-8 for preparing a peptide for use as a medicament.

## Revendications (Revendications pour l'(les) Etat(s) contractant(s) suivant(s) : BE, CH, LI, DE, FR, GB, IT, NL, SE)

1. Peptide de formule 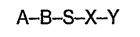 dans laquelle
A représente l'arginine, la lysine, l'ornithine ou l'homoarginine, chacune en configuration L ou D, un groupe co-amino, ω-guanidino- ou ω-diméthyl- aminoalcanoyle ayant de 3 à 6 atomes de carbone et éventuellement un groupe a-amino en configuration D ou L, qui de son côté peut porter un groupe atcanoyfe ayant de 1 à 6 atomes de carbone, aroyle ayant de 7 à 11 atomes de carbone, cycloalcanoyle ayant jusqu'à 2 atomes de carbone dans la partie alcoyle et de 5 à 7 atomes de carbone dans la partie cycloalcoyle, un groupe aralca- noyle ayant au total jusqu'à 9 atomes de carbone, auquel cas un groupe -CH₂- peut être remplacé par-O-ou-S-, un groupe alcoyl- ou aralcoyl-oxycarbonyle ayant jusqu'à 7 atomes de carbone ou un groupe succinoyle, succinamoyle, glutaroyle, glutaminyle, pyroglutamyle, phtaloyle, phtalamidyle ou 2-carboxybenzoyle,
B représente un acide aminé basique, de préférence la L-lysine, la L-arginine, la L-homoarginine ou la L-ornithine
X représente la L-valine ou la L-isoleucine et
Y représente un acide aminé de configuration L ou D, avec une chaîne latérale hydrophobe, dont la partie ester, amide, alcoylamide a de 1 à 6 atomes de carbone ou la partie aralcoylamide a de 7 à 10 atomes de carbone, un groupe alcoylamide ou alcoylester ayant de 1 à 6 atomes de carbone ou un groupe aralcoylamide ou aralcoylester ayant de 7 à 10 atomes de carbone,

S est Glu, D-Glu, D-Asp ou l'acide D-a-amino- adipique, et de leurs sels.

2. Peptide de formule 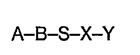 dans laquelle
A représente l'arginine, la lysine, l'ornithine ou l'homoarginine, chacune en configuration L ou D, un groupe co-amino-, ω-guanidino- ou ω-diméthyl- aminoalcanoyle ayant de 3 à 6 atomes de carbone et éventuellement un groupe a-amino en configuration D ou L qui, de son côté, peut porter un groupe alcanoyle ayant de 1 à 6 atomes de carbone, aroyle ayant de 7 à 11 atomes de carbone, cycloalcanoyle ayant jusqu'à 2 atomes de carbone dans la partie alcoyle et de 5 à 7 atomes de carbone dans la partie cycloalcoyle, un groupe aral- canoyle ayant jusqu'à 9 atomes de carbone au total, auquel cas un groupe -CH₂- peut être remplacé par -0- ou -S-, un groupe alcoyl- ou aralcoyl-oxycarbonyle ayant jusqu'à 7 atomes de carbone, ou un groupe succinoyle, succinamoyle, glutaroyle, glutaminyle, pyroglutamyle, phtaloyle, phtalamidyle ou 2 carboxybenzoyle,
B représente un acide aminé basique, de préférence la L-lysine, la L-arginine, la L-homoarginine ou la L-ornithine,
X représente la L-valine ou la L-isoleucine et
Y est un acide L- ou D-aminé avec une chaîne latérale hydrophobe dont la partie ester, amide, alcoylamide a de 1 à 6 atomes de carbone ou la partie aralcoylamide a de 7 à 10 atomes de carbone, un groupe alcoylamide ou alcoylester ayant de 1 à 6 atomes de carbone ou un groupe aralcoylamide ou aralcoylester ayant de 7,à10 atomes de carbone,
S est Glu, et de leurs sels.

3. Peptide de formule 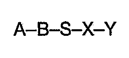 dans laquelle
A représente l'arginine, la lysine, l'ornithine ou t'homoarginine, chacune en configuration L ou D, un groupe ω-amino, ω-guanidino- ou ω-diméthyl- aminoalcanoyle ayant de 3 à 6 atomes de carbone et éventuellement un groupe a-amino en configuration D ou L, qui de son côté peut porter un groupe alcanoyle ayant de 1 à 6 atomes de carbone, aroyle ayant de 7à11 atomes de carbone, cycloalcanoyle ayant jusqu'à 2 atomes de carbone dans la partie alcoyle et de 5 à 7 atomes de carbone dans la partie cycloalcoyle, un groupe aral- canoyle ayant au total jusqu'à 9 atomes de carbone, auquel cas un groupe -CH₂- peut être remplacé par -0- ou -S-, un groupe alcoyl- ou aralcoyl-oxycarbonyle ayant jusqu'à 7 atomes de carbone, ou un groupe succinoyle, succinamoyle, glutaroyle, glutaminyle, pyroglutamyle, phtaloyle, phtalamidyle ou 2-carboxybenzoyle,
B représente un acide aminé basique, de préférence la L-lysine, la L-arginine, la L-homoarginine ou la L-ornithine,
X représente la L-valine ou la L-isoleucine et
X représente un acide L- ou D-aminé avec une chaîne latérale hydrophobe dont la partie ester, amide ou alcoylamide a de 1 à 6 atomes de carbone ou la partie aralcoylamide a de 7 à 10 atomes de carbone, un groupe alcoylamide ou alcoylester ayant de 1 à 6 atomes de carbone, ou un groupe aralcoylamide ou aralcoylester ayant de 7 à 10 atomes de carbone,
S est D-Glu, D-Asp ou l'acide D-a-aminoadipi- que, auquel cas sont exclus des peptides de formule A-Lys-S-X-Y et A-D-Lys-S-X-Y, dans lesquels A représente l'arginine éventuellement substituée par un groupe alcanoyle en C,-C₆ ou par Gin, l'homoarginine ou un groupe ω-guanidino-alcanoyle en C₃-C₆, et
Y représente simultanément Tyr ou D-Tyr, ainsi que leurs amides, alcoylamides en C,-C₆ ou leurs ester d'alcoyle en C₁-C₆; et de leurs sels.

4. Peptide selon l'une des revendications 1 et 2, de formule Arg-Lys-Glu-Val-Trp-OMe.

5. Peptide selon l'une des revendications 1 et 2, de formule Arg-Lys-Glu-Val-Tyr-OMe.

6. Peptide selon l'une des revendications 1 et 2, de formule D-Lys-Arg-Glu-Val-Tyr-OMe.

7. Peptide selon la revendication 1, de formule Arg-Lys-D-Aad-Val-Tyr-OMe.

8. Peptide selon la revendication 1, de formule Arg-Lys-D-Glu-Val-Tyr-OMe.

9. Procédé de préparation de peptides selon l'une quelconque des revendications 1 à 8, caractérisé en ce qu'on construit des séquences d'acides aminés de formule 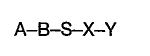 dans laquelle A, B, S, X et Y ont les significations indiquées dans les revendications 1 à 8, selon des procédés de la synthèse des peptides.

10. Peptide selon l'une quelconque des revendications 1à8 pour utilisation comme agent pour influencer la maturation des lymphocytes T.

11. Peptide selon l'une quelconque des revendications 1à8 pour utilisation comme médicament.

## Revendications (Revendications pour l'(les) Etat(s) contractant(s) suivant(s) : Autriche)

1. Procédé de préparation de nouveaux peptides de formule générale 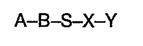 dans laquelle
A représente l'arginine, la lysine, l'ornithine ou l'homoarginine, chacune en configuration L ou D, un groupe ω-amino-, ω-guanidino- ou ω-diméthyl- aminoalcanoyle ayant de 3 à 6 atomes de carbone et éventuellement un groupe a-amino en configuration D ou L, qui de son côté peut porter un groupe alcanoyle ayant de 1 à 6 atomes de carbone, aroyle ayant de 7 à 11 atomes de carbone, cycloalcanoyle ayant jusqu'à 2 atomes de carbone dans le groupe alcoyle et de 5 à 7 atomes de carbone dans le groupe cycloalcoyle, un groupe aral- canoyle ayant au total jusqu'à 9 atomes de carbone, auquel cas un groupe -CH₂- peut être remplacé par -O- ou par -S-, un groupe alcoyl- ou aralcoyl-oxycarbonyle ayant jusqu'à 7 atomes de carbone, ou un groupe succinoyle, succinamoyle, glutaroyle, glutaminyle, pyroglutamyle, phtaloyle, phtalamidyle ou 2-carboxybenzoyle,
B représente un acide aminé basique, de préférence la L-lysine, la L-arginine, la L-homoarginine ou la L-ornithine,
X représente la L-valine ou la L-isoleucine et
Y représente un acide L- ou D-aminé avec une chaîne latérale hydrophobe dont la partie ester, amide, alcoylamide a de 1 à 6 atomes de carbone et la partie aralcoylamide a de 7 à 10 atomes de carbone, un groupe alcoylamide ou alcoylester ayant de 1 à 6 atomes de carbone ou un groupe aralcoylamide ou aralcoylester ayant de 7 à 10 atomes de carbone,
S est Glu- D-Glu, D-Asp ou l'acide D-a-amino- adipique, et de leurs sels, caractérisé en ce qu'on construit des séquences d'acides aminés de formule indiquée selon des procédés de la synthèse des peptides.

2. Procédé de préparation de nouveaux peptides de formule générale 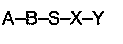 dans laquelle
A représente l'arginine, la lysine, l'ornithine ou l'homoarginine, chacune en configuration L ou D, un groupe co-amino-, co-guanidino- ou w-diméthyl- aminoalcanoyle ayant de 3 à 6 atomes de carbone et éventuellement un groupe a-amino en configuration D ou L, qui de son côté peut porter un groupe alcanoyle ayant de 1 à 6 atomes de carbone, aroyle ayant de 7 à 11 atomes de carbone, cycloalcanoyle ayant jusqu'à 2 atomes de carbone dans la partie alcoyle et de 5 à 7 atomes de carbone dans la partie cycloalcoyle, un groupe aral- canoyle ayant jusqu'à 9 atomes de carbone au total, auquel cas un groupe -CH₂- peut être remplacé par -O- ou -S-, un groupe alcoyl- ou aralcoyl-oxycarbonyle ayant jusqu'à 7 atomes de carbone, ou un groupe succinoyle, succinamoyle, glutaroyle, glutaminyle, pyroglutamyle, phtaloyle, phtalamidyle ou 2-carboxybenzoyle,
B représente un acide aminé basique, de préférence la L-lysine, la L-arginine, la L-homoarginine ou la L-ornithine,
X représente la L-valine ou la L-isoleucine et
Y représente un acide L- ou D-aminé avec une chaîne latérale hydrophobe dont la partie ester, amide, alcoylamide a de 1 à 6 atomes de carbone ou la partie aralcoylamide a de 7 à 10 atomes de carbone, un groupe alcoylamide ou alcoylester ayant de 1 à 6 atomes de carbone ou un groupe aralcoylamide ou aralcoylester ayant de 7 à 10 atomes de carbone,
S est Glu, et de leurs sels, lequel procédé est caractérisé en ce qu'on construit des séquences d'acides aminés de formule indiquée selon des procédés de la synthèse des peptides et on convertit éventuellement ces peptides en leurs sels.

3. Procédé de préparation de nouveaux peptides de formule générale 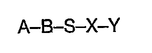 dans laquelle
A représente l'arginine, la lysine, l'ornithine ou l'homoarginine chacune en configuration L ou D, un groupe co-amino-, w-guanidino- ou ω-diméthyl- aminoalcanoyle ayant de 3 à 6 atomes de carbone et éventuellement un groupe a-amino en configuration D ou L, qui de son côté peut porter un groupe alcanoyle ayant de 1 à 6 atomes de carbone, aroyle ayant de 7 à 11 atomes de carbone, cycloalcanoyle ayant jusqu'à 2 atomes de carbone dans la partie alcoyle et de 5 à 7 atomes de carbone dans la partie cycloalcoyle, un groupe aral- canoyle ayant au total jusqu'à 9 atomes de carbone, auquel cas un groupe -CH₂- peut être remplacé par -0- ou -S-, un groupe alcoyl- ou aralcoyl-oxycarbonyle ayant jusqu'à 7 atomes de carbone, ou un groupe succinoyle, succinamoyle, glutaroyle, glutaminyle, pyroglutamyle, phtaloyle, phtalamidyle, ou 2-carboxybenzoyle,
B représente un acide aminé basique, de préférence la L-lysine, la L-arginine, la L-homoarginine ou la L-ornithine,
X représente la L-valine ou la L-isoleucine et
Y représente un acide L- ou D-aminé avec une chaîne latérale hydrophobe dont la partie ester, amide, alcoylamide a de 1 à 6 atomes de carbone ou la partie aralcoylamide a de 7 à 10 atomes de carbone, un groupe alcoylamide ou alcoylester ayant de 1à6 atomes de carbone ou un groupe aralcoylamide ou aralcoylester ayant de 7 à 10 atomes de carbone,
S est D-Glu, D-Asp ou l'acide D-a-aminoadipi- que, auquel cas le procédé de préparation de peptides de formules A-Lys-S-X-Y et A-D-Lys-S-X-Y dans lesquels A représente l'arginine éventuellement substituée par un groupe alcanoyle en C₁-C₆ ou par Gin, l'homoarginine ou un groupe co-guanidino-alcanoyle en C₃-C₆ et Y représente simultanément Tyr ou D-Tyr, ainsi que leurs amides, alcoylamides en C₁-C₆ ou alcoylesters en C₁-C₆, est exclu, et de leurs sels, lequel procédé est caractérisé en ce qu'on construit des séquences d'acides aminés de formule indiquée selon des procédés de la synthèse des peptides et on convertit éventuellement ces peptides en leurs sels.

4. Procédé selon l'une des revendications 1 et 2, caractérisé en ce qu'on prépare Arg-Lys-Glu-Val-Trp-OMe.

5. Procédé selon l'une des revendications 1 et 2, caractérisé en ce qu'on prépare Arg-Lys-Glu-Val-Tyr-OMe.

6. Procédé selon l'une des revendications 1 et 2, caractérisé en ce qu'on prépare D-Lys-Arg-Glu-Val-Tyr-OMe.

7. Procédé selon la revendication 1, caractérisé en ce qu'on prépare Arg-Lys-D-Aad-Val-Tyr-OMe.

8. Procédé selon la revendication 1, caractérisé en ce qu'on prépare Arg-Lys-D-Glu-Val-Tyr-OMe.

9. Procédé selon l'une quelconque des revendications 1 à 8, pour la préparation d'un peptide pour utilisation comme agent pour influencer la maturation des lymphocytes T.

10. Procédé selon l'une quelconque des revendications 1 à pour la préparation d'un peptide utilisable comme médicament.
